(19) Europäisches Patentamt
European Patent Office
Office européen des brevets

(11) EP 3 741 389 A1

# (12) EUROPEAN PATENT APPLICATION

(43) Date of publication:
**25.11.2020 Bulletin 2020/48**

(21) Application number: **20176176.4**

(22) Date of filing: **22.05.2020**

(51) Int Cl.:
*A61K 39/395* (2006.01) *A61P 9/00* (2006.01)
*A61P 11/00* (2006.01) *A61P 21/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.05.2019 US 201962852227 P**
**18.06.2019 US 201962863143 P**

(71) Applicant: **Fibrogen, Inc.**
**San Francisco, CA 94158 (US)**

(72) Inventors:
- **ELMANKABADI, Bassem**
  **San Francisco, CA California 94158 (US)**
- **YU, Kin-Hung Peony**
  **San Francisco, CA California 94158 (US)**
- **KOUCHAKJI, Elias**
  **San Francisco, CA California 94158 (US)**
- **SEKAYAN, Tro**
  **San Francisco, CA California 94158 (US)**

(74) Representative: **Wise, Daniel Joseph**
**Carpmaels & Ransford LLP**
**One Southampton Row**
**London WC1B 5HA (GB)**

## (54) A CONNECTIVE TISSUE GROWTH FACTOR (CTGF) INHIBITOR FOR USE IN THE TREATMENT OF MUSCULAR DYSTROPHIES

(57) The invention relates to methods and agents useful for treating muscular dystrophies (MDs), in particular, Duchenne muscular dystrophy (DMD). Methods and agents for treating various physiological and pathological features associated with muscular dystrophies are also provided.

EP 3 741 389 A1

## Description

This application claims the benefit of U.S. Provisional Application Serial No. 62/863,143, filed on 18 June 2019, and U.S. Provisional Application Serial No. 62/852,227, filed on 23 May 2019, incorporated by reference in their entirety.

## FIELD OF THE INVENTION

The invention relates to methods and agents useful for treating muscular dystrophies (MDs), in particular, Duchene muscular dystrophy (DMD). Methods and agents for treating various physiological and pathological features associated with muscular dystrophies are also provided.

## BACKGROUND

Muscular dystrophy refers to a group of more than 30 hereditary muscle diseases characterized by defects in certain muscle proteins leading to progressive skeletal muscle weakness, degeneration of skeletal muscle fibers and death of muscle cells and tissue. Duchenne muscular dystrophy is the most common form of MD and results from mutations in the dystrophin gene (locus Xp21.2). Dystrophin is an important structural component in muscle tissue. A lack of functional dystrophin leads to progressive muscular damage, degeneration and weakness, associated with motor delays, loss of ambulation, respiratory and cardiac dysfunctions. DMD is typically inherited in an X-linked recessive fashion, but it can occur because of spontaneous mutations. The worldwide prevalence of DMD is estimated to be 4.78 per 100,000 males, with an incidence of 10.7 to 27.7 per 100,000 (Mah et al., Neuromuscul Disord. 2014;24(6):482-491).

Historically, loss of ambulation occurred before 12 years of age in DMD patients, but improved standards of care has delayed the loss by 2-5 years (Andrews et al. Adolesc Health Med Ther. 2018;9:53-63). Typically, DMD patients become wheelchair bound before developing significant respiratory and cardiac muscle weakness. Progressive respiratory muscle weakness leads to hypoventilation and/or recurrent atelectasis and pneumonia, secondary to decreased cough effectiveness (McKim et al. Arch Phys Med Rehabil. 2012;93(7):1117-1122). Corticosteroid use can delay by 2- 3 years, a pathological decline in forced vital capacity % predicted (FVCpp) (Henricson et al. Muscle Nerve. 2013;48(1):55-67), but once patients are in the decline phase, glucocorticoid treated patients have a similar rate of decline as untreated patients (Mayer et al. Pediatr Pulmonol. 2015;50(5):487-494; Finder et al. Am J Respir Crit Care Med. 2017;196(4):512-519).

Corticosteroid use can also delay the onset of cardiomyopathy (Barber BJ et al. J Pediatr. 2013 Oct;163(4):1080-4) with a longer treatment duration correlated with a smaller age related increase in myocardial fibrosis burden (Tandon et al. J Am Heart Assoc. 2015;4:(4):3001338). Despite these advances, cardiac dysfunction is the leading cause of morbidity and mortality in DMD patients (Schram et al. JAm Coll Cardiol. 2013;61(9):948--954). Although the clinical course of cardiac and skeletal muscle involvement can be variable, death usually occurs because of cardiac or respiratory compromise.

Given the dire clinical course of the disease, additional treatments are needed, particularly treatments that address the decline in pulmonary and cardiac functions. The present invention meets these needs.

## SUMMARY OF THE INVENTION

In one aspect of the invention, a method is provided for treating a muscular dystrophy (MD). The method comprises administering to a subject in need thereof, an effective amount of an agent that inhibits connective tissue growth factor (CTGF), thereby treating the MD. In some embodiments, the MD is selected from the group consisting of Duchenne muscular dystrophy, Becker's muscular dystrophy, congenital muscular dystrophy, distal muscular dystrophy, Emery-Dreifuss muscular dystrophy, facioscapulohumeral muscular dystrophy, limb-girdle muscular dystrophy, myotonic muscular dystrophy, and oculopharyngeal muscular dystrophy. In particular embodiments, the MD is Duchenne muscular dystrophy. In some embodiments, the subject is non-ambulatory.

In a further aspect of the invention, a method is provided for improving pulmonary function in a subject with a muscular dystrophy. The method comprises administering to a subject in need thereof an effective amount of an agent that inhibits CTGF, thereby improving the subject's pulmonary function.

In some embodiments, the improvement in pulmonary function is a reduction in the rate of decline, a stabilization in the rate of decline or a reversal (improvement) in the rate of decline of the subject's forced vital capacity % predicted (FVCpp), forced expiratory volume at 1 second % predicted (FEV1pp), peak expiratory flow rate % predicted (PEFRpp), maximum static inspiratory pressure % predicted (MIPpp) or maximum static expiratory pressure % predicted (MEPpp).

In an additional aspect of the invention, a method is provided for improving cardiac function in a subject with a muscular dystrophy. The method comprises administering to a subject in need thereof an effective amount of an agent that inhibits CTGF, thereby improving the subject's cardiac function. In some embodiments, the improvement in cardiac function is a reduction in the rate of decline, a stabilization in the rate of decline or a reversal (improvement) in the rate of decline of the subject's left ventricular ejection fraction percentage (LVEF%) circumferential peak strain (%), or global circumferential strain (%).

In a further aspect of the invention, a method is provided for reducing the development of or reducing the progression rate of cardiac fibrosis in a subject with a muscular dystrophy. The method comprises administering to a subject in need thereof an effective amount of an anti-CTGF agent, thereby reducing the development of or reducing the progression rate of cardiac fibrosis. In some embodiments, the subject's degree of cardiac fibrosis is assessed by MRI using mass of late gadolinium enhancement.

In another aspect of the invention, a method is provided for of increasing the muscle strength of a subject with a muscular dystrophy. The method comprises administering to a subject in need thereof an effective amount of an anti-CTGF agent, thereby increasing the subject's muscle strength. In some embodiments, the increased muscle strength is measured as an increase in the subject's grip.

In an additional aspect of the invention, a method is provided for reducing muscle inflammation, muscle edema, fat infiltration or percentage of dystrophic muscle composition in a subject with a muscular dystrophy. The method comprises administering to the subject an effective amount of an anti-CTGF agent, thereby reducing muscle inflammation, muscle edema, fat infiltration or percentage of dystrophic muscle composition in the subject.

These and other methods of the invention are accomplished by administering an anti-CTGF agent to the subject with a MD. In some embodiments, the anti-CTGF agent is selected from group consisting of anti-CTGF antibodies, anti-CTGF antibody fragments, anti-CTGF antibody mimetics and anti-CTGF oligonucleotides. In further embodiments, the anti-CTGF agent is an anti-CTGF antibody. In additional embodiments, the anti-CTGF antibody is a human or humanized antibody. In particular embodiments, the anti-CTGF antibody is pamrevlumab. In other embodiments, the anti-CTGF antibody binds to CTGF competitively with pamrevlumab. In a specific embodiment, the anti-CTGF antibody is identical to CLN1 or mAb1 as described in U.S. Patent No. 7,405,274. In specific embodiments, the effective amount of an anti-CTGF antibody is at least 35 mg/kg.

In some embodiments, the anti-CTGF oligonucleotide is selected from the group consisting of antisense oligonucleotides, siRNAs, miRNAs and shRNAs. In certain embodiments, the anti-CTGF agent is used in combination with another therapeutic modality. In particular embodiments, the anti-CTGF agent is used in combination with a corticosteroid, ataluren and/or eteplirsen.

These and other embodiments of the invention will readily occur to those of skill in the art in light of the disclosure herein, and all such embodiments are specifically contemplated. Each of the limitations of the invention can encompass various embodiments of the invention. It is, therefore, anticipated that each of the limitations of the invention involving any one element or combinations of elements can be included in each aspect of the invention. This invention is not limited in its application to the details of construction and the arrangement of components set forth in the following description or illustrated in the drawings. The invention is capable of other embodiments and of being practiced or of being carried out in various ways.

## BRIEF DESCRIPTION OF THE DRAWINGS

Figure 1 is a waterfall plot of the change from baseline at Week 48 in FVCpp in 19 non-ambulatory subjects with DMD following treatment with pamrevlumab.

Figure 2 is a bar plot comparing the LS mean change from baseline at 1-year in FVCpp seen in the current study to historic controls (Ricotti et al. Neuromuscul Disord. 2019;29(4):261-268).

Figure 3 is a waterfall plot of the change from baseline at Week 48 in FEV1pp in 18 non-ambulatory subjects with DMD following treatment with pamrevlumab.

Figure 4 is a bar plot comparing the LS mean change from baseline at 1-year in FEVlpp seen in the current study to the change observed in historic placebo treated non-ambulatory subjects and glucocorticosteroid treated non-ambulatory subjects (Meier et al. Neuromuscl Disord. 2016;27:307-314).

Figure 5 is a waterfall plot of the change from baseline at Week 48 in PEFRpp in 19 non-ambulatory subjects with DMD following treatment with pamrevlumab.

Figure 6 is a bar plot comparing the LS mean change from baseline at 1-year in PEFRpp seen in the current study to the change observed in historic controls (Ricotti et al. *supra).*

Figure 7 is a waterfall plot of the change from baseline at 1-year in LVEF in 19 non-ambulatory subjects with DMD following treatment with pamrevlumab.

Figure 8 is a bar plot comparing the LS mean change from baseline at 1-year in LVEF% seen in the current study to the change observed during the natural course of DMD (McDonald et al. Lancet. 2018;391(10119):451-461).

Figure 9 is a waterfall plot of change from baseline at 1-year in mean circumferential peak strain (%) in 14 subjects with DMD following treatment with pamrevlumab.

Figure 10 is a waterfall plot of change from baseline at 1-year in global circumferential peak strain (%) in 14 subjects with DMD following treatment with pamrevlumab

Figure 11 is a waterfall plot of the change from baseline at 1-year in cardiac fibrosis score as measured by mean mass of late gadolinium enhancement (MLGE) in 18 non-ambulatory subjects with DMD following treatment with pamrevlumab.

Figure 12 is a scatter plot and Spearman correlation of changes from baseline at 1-year in LVEF (%) versus change in cardiac fibrosis score (MLGE) at 1-year for 18 non-ambulatory subjects with DMD following treatment with pamrevlumab.

Figures 13A and 13B are waterfall plots of the change from baseline at Week 48 in grip strength for dominant and non-dominant hands, respectively, in 19 non-ambulatory subjects with DMD following treatment with pamrevlumab.

Figure 14 is a bar plot comparing the LS mean change from baseline at 1-year in grip strength for non-dominant and dominant hands, respectively, seen in the current study to the changes observed during the natural course of non-ambulatory DMD patients (Seferian et al. PLoS One. 2015;10(2):e0113999).

Figure 15 is a waterfall plot of the change from baseline at Week 48 in performance of the upper limb (PUL) assessment from 19 non-ambulatory subjects with DMD following treatment with pamrevlumab.

Figure 16 is a bar plot comparing the LS mean change from baseline at 1-year in PUL assessment for 19 non-ambulatory subjects seen in the current study to the changes observed in published data (Ricotti et al. Neuromuscul Disord. 2019;29(4): 261-268).

Figure 17 is a waterfall plot of the change from baseline at 1-year in the degree of inflammation, edema, dystrophic muscle composition and fat infiltration of the upper arms of 11 non-ambulatory subjects with DMD following treatment with pamrevlumab. Tissue composition was determined through T2-mapping using MRI and computer-aided detection (CAD) assessment.

Figure 18 is a bar plot comparing the LS mean change from baseline at 1-year in upper arm fat fraction (%) seen in the current study (n=8) to the changes observed during the natural course of non-ambulatory DMD patients (n=15) (Hogrel JY et al, Neurology. 2016;86(11):1022-30).

Figure 19 is a scatter plot and Spearman correlation of changes from baseline at 1-year in upper arm biceps brachii T2-mapping compared to change in PUL total score at 1-year for 11 non-ambulatory subjects with DMD following treatment with pamrevlumab.

## DESCRIPTION OF THE INVENTION

It is to be understood that the invention is not limited to the particular methodologies, protocols, cell lines, assays, and reagents described herein, as these may vary. It is also to be understood that the terminology used herein is intended to describe particular embodiments of the invention, and is in no way intended to limit the scope of the invention as set forth in the appended claims.

Each of the limitations of the invention can encompass various embodiments of the invention. It is, therefore, anticipated that each of the limitations of the invention involving any one element or combinations of elements can be included in each aspect of the invention. This invention is not limited in its application to the details of construction and the arrangement of components set forth in the following description or illustrated in the drawings. The invention is capable of other embodiments and of being practiced or of being carried out in various ways.

It must be noted that as used herein and in the appended claims, the singular forms "a," "an," and "the" include plural references unless context clearly dictates otherwise. Thus, for example, a reference to "an anti-CTGF oligonucleotide" includes a plurality of such anti-CTGF oligonucleotides; a reference to "an antibody" is a reference to one or more antibodies and to equivalents thereof known to those skilled in the art, and so forth.

Unless defined otherwise, all technical and scientific terms used herein have the same meanings as commonly understood by one of ordinary skill in the art to which this invention belongs. Although any methods and materials similar or equivalent to those described herein can be used in the practice or testing of the invention, the preferred methods, devices, and materials are now described. All publications cited herein are incorporated herein by reference in their entirety for the purpose of describing and disclosing the methodologies, reagents, and tools reported in the publications that might be used in connection with the invention. Nothing herein is to be construed as an admission that the invention is not entitled to antedate such disclosure by virtue of prior invention.

The practice of the invention will employ, unless otherwise indicated, conventional methods of chemistry, biochemistry, molecular biology, cell biology, genetics, immunology and pharmacology, within the skill of the art. Such techniques are explained fully in the literature. See, e.g., Gennaro, AR, ed. Remington's Pharmaceutical Sciences, 18th ed. Mack Publishing Co. (1990); Colowick, S et al., eds., Methods In Enzymology, Academic Press, Inc.; Handbook of Experimental Immunology, Vols. I-IV, DM Weir and CC Blackwell, eds., Blackwell Scientific Publications (1986); Maniatis, T. et al., eds. Molecular Cloning: A Laboratory Manual, 2nd edition, Vols. I-III, Cold Spring Harbor Laboratory Press (1989); Ausubel, F. M. et al., eds. Short Protocols in Molecular Biology, 4th edition, John Wiley & Sons (1999); Ream et al., eds. Molecular Biology Techniques: An Intensive Laboratory Course, Academic Press (1998); PCR (Introduction to Biotechniques Series), 2nd ed. Newton & Graham eds., Springer Verlag (1997).

**Definitions**

As used herein, the term "about" refers to ± 10 % of the numerical value of the number with which it is being used. Therefore, about 50% means in the range of 45%-55%.

Also, the phraseology and terminology used herein is for the purpose of description and should not be regarded as limiting. The use of "including," "comprising," or "having," "containing", "involving", and variations thereof herein, is meant to encompass the items listed thereafter and equivalents thereof as well as additional items.

As used herein, the term "subject," "individual," and "patient" are used interchangeably to refer to a mammal. In a preferred embodiment, the mammal is a primate, and more preferably a human being.

As used herein, the term "muscular dystrophy" or "MD" describes a degenerative muscular disorder characterized by progressive loss of muscle function leading to atrophy and/or spasticity of the associated musculature. Muscular dystrophies include Becker muscular dystrophy, congenital muscular dystrophy, Duchenne muscular dystrophy, distal muscular dystrophy, Emery-Dreifuss muscular dystrophy, facioscapulohumeral muscular dystrophy, limb-girdle muscular dystrophy, myotonic muscular dystrophy, and oculopharyngeal muscular dystrophy. In particular embodiments, the MD is Duchenne muscular dystrophy. The methods and agents of the invention may be used to treat any form of MD.

The terms, "treat," "treating" and "treatment," as used herein, refer to the administration of a therapeutic agent (e.g., anti-CTGF agent) to the subject in need thereof, in order to achieve a beneficial effect including changes in a pathological feature of a cell type, tissue or organ affected by MD; the prevention or reduction of one or more symptoms of a MD; improvement in the prognosis of the subject; or the extension of survival of the subject. Treating a MD may prevent or delay the development of the loss of mobility, arrest the physical decline of the subject, stabilize the disease, reduce the need for medication or supportive measures, extend the period of independent living or freedom from the need for ventilator assistance, increase the time to needing a tracheostomy or increase the survival of the subject.

As used herein, the terms "reduce," "reducing" or "reduction" in the context of treating a subject with MD refers to treatment that eases, mitigates, alleviates, ameliorate or decreases the effect or severity of a symptom of the MD, e.g., DMD, without curing the disease. Any indicia of success in reducing a symptom of a MD is recognized as reducing the symptom. The reduction of a MD symptom can be determined using standard routine clinical tests, observations and questionnaires that are well within the skill and knowledge of a medical professional. Non-limiting exemplary tests can include blood tests, e.g., serum creatine kinase or serum aldolase, muscle biopsies, genetic testing, neurologic testing, cardiac testing, e.g., electrocardiogram, exercise testing, imaging tests, such as magnetic resonance imaging (MRI) or ultrasound; observations made during a physical examination; as well as patient self-assessments and quality of life questionnaires.

When used in the context of the progression of a MD, the terms "reduce," "reducing" and "reduction" refer to slowing of the progression of the disease or a disease symptom. In some embodiments, the methods of the invention reduce the progression of a MD or a MD symptom by at least 1 month, at least 2 months, at least 3 months, at least 4 months, at least 5 months, at least 6 months, at least 8 months, at least 10 months, at least 12 months, at least 15 months, at least 18 months or at least 2 years, at least 3 years or at least 4 years compared to a control group or a historical control(s).

The terms "reduce," "reducing" and "reduction" when used in the context to a disease symptom refer to the moderation, attenuation or diminution of the symptom. In other embodiments, the methods of the invention reduce the severity of a MD symptom by at least 10%, at least 15%, at least 20%, at least 25%, at least 30%, at least 35%, at least 40%, at least 50%, at least 60%, at least 70%, at least 80% or at least 90% compared to a control group or historical controls.

In specific embodiments, the symptoms for which treatment with an anti-CTGF delays the clinical presentation, or reduces a MD symptom includes the muscle weakness, muscle fatigue, muscle cramps, muscle or joint pain, loss of control of voluntary muscle movement, loss of ambulation, difficulty breathing, including hypoventilation, the development of atelectasis, the development of pneumonia, or the development of cardiomyopathy.

In some embodiments, treatment of a subject with a MD with an effective amount of an anti-CTGF agent increases the survival of the MD patient. In further embodiments, the administration of an effective amount of an anti-CTGF agent increases the survival of a MD patient by at least 10%, 20%, 30%, 40%, 50%, 60%, 70%, 80%, 90% or 100% compared to a control group or a historical control(s). In other embodiments, the administration of an effective amount of an anti-CTGF agent increases survival of a MD patient by at least at least 3 months, at least 6 months, at least 9 months, at least 12 months, at least 18 months, at least 24 months, at least 3 years, at least 4 years, or at least 5 years compared to a control group or a historical control(s).

Subjects with muscular dystrophies, particularly, DMD, experience disease related declines in pulmonary function. In contrast to normal boys that exhibit an increase in lung capacity over time with increased age, in a study of predominantly corticosteroid-treated DMD patients, both forced vital capacity (FVC) and peak expiratory flow (PEF), measured in liters per minute, changed in three phases with increasing age (Mayer et al. *supra*). Up to approximately age 10 years, subjects showed a nearly linear increase in FVC and PEF, followed by a period of no increase through 16 years, after which there was a rapid decline. However, FVCpp and PEFRpp declined almost linearly from the 6 to 8 years of age cohort through the 16 to 18 years of age cohort (Moxley et al. Neurology. 2005;64:13-20). There was an annual rate of change for FVCpp of -5.0 $\pm$ 0.7%/yr, and for PEFRpp, the rate of change was -5.8 $\pm$ 0.6%/yr (mean$\pm$ SE) (Moxley RT III et al. *supra*).

Numerous measurements to access pulmonary function are known in the art including the following:

Vital capacity (VC) is the total volume of air that can be moved in and out of the lungs. VC is equal to the combined inspiratory reserve volume, tidal volume, and expiratory reserve volume.

Forced vital capacity (FVC) is the vital capacity from a maximally forced expiratory effort.

FVCpp is a subject's measured FVC expressed as the percentage of the predicted FVC for the subject. As used herein, all FVCpp values are absolute values and not relative values.

Residual volume (RV) is the volume of air remaining in the lungs after a maximal exhalation.

Forced expiratory volume (FEV) is the expiratory volume of air from a maximally forced expiratory effort, usually measured over a set period of time, e.g., 1 second, FEV1; 6 seconds, FEV6; etc.

Forced inspiratory flow (FIF) is the inspiratory volume of air from a maximally forced inspiratory effort, usually measured over a set period of time, e.g., 1 second, FIF1; 6 seconds, FIF6; etc.

Peak expiratory flow (PEF) rate is the highest forced expiratory flow rate.

Inspiratory reserve volume (IRV) is the maximal volume that can be inhaled after a normal inspiration, measured from the end-inspiratory level.

Tidal volume (TV) is the volume of air inhaled or exhaled during one respiratory cycle, typically measured at rest.

Inspiratory capacity (IC) is the sum of the inspiratory reserve volume and the tidal volume.

Functional residual capacity (FRC) is the sum of the expiratory reserve volume and the residual volume. Typically, FRC represents the volume of air in the lungs at the end of a normal expiration.

Total lung capacity (TLC) is the sum of the vital capacity and residual volume that represents the total volume of air that can be contained in the lung.

Expiratory reserve volume (ERV) is the maximal volume of air that can be exhaled after a normal expiration, measured from the end-expiratory position.

Maximum voluntary ventilation (MVV) is the volume of air expired in a specified time period during repetitive maximal effort.

FEV1/FVC ratio means the ratio between forced expiratory volume in one second and forced vital capacity.

Many of these pulmonary function measurements can be readily obtained through the use of a spirometer as is well-known in the art. Residual volume can be obtained through indirect methods such as radiographic planimetry, body plethysmography, closed circuit dilution (including the helium dilution technique), and nitrogen washout.

The abnormal change in pulmonary function seen in the subjects with DMD, termed herein as a "pathologic change in pulmonary function," is a degree of change that represents at least a 5% difference in the subject's measured pulmonary function compared to a baseline measurement from the subject or to a matched control value, i.e., normal person of similarly matched race or ethnicity, gender, age, height, and weight. Any of the pulmonary function measurements described above can be associated with a pathological change. In some embodiments, a pathologic change in pulmonary function is at least a 5%, 10%, 15%, 20%, 25%, 30%, 40%, 50%, 60%, 70%, 80%, 90%, 100%, 125%, 150%, 200%, 300%, 400%, 500%, 600%, 700%, 800% or 1000% difference compared to a baseline measurement from the subject or compared to the pulmonary function value for a matched control. In some embodiments, the baseline measurement is taken, for example, 4 weeks, 8 weeks, 12 weeks, 24 weeks, 36 weeks, 48 weeks, or 12 months prior to the subsequent measurement.

In some embodiments, the pathological change in pulmonary function is a change in FVC. In particular embodiments, the pathologic change in a subject's FVC when compared to a subject's baseline measurement or compared to a matched control, is a decrease of at least about -0.05 liters, -0.10 liters, -0.15 liters, -0.20 liters or -0.25 liters. In further embodiments, a method is provided for increasing FVC in a subject with MD by administering an effective amount of an anti-CTGF antibody. In additional embodiments, treatment with an effective amount of an anti-CTGF antibody increases FVC by at least 0.05 liters, 0.1 liters, 0.15 liters, 0.20 liters, 0.25 liters or 0.3 liters compared to a subject's baseline FVC or to a historical control(s).

In some embodiments, the pathologic change in pulmonary function is the change in FVCpp compared to a subject's baseline measurement or compared to a matched control, wherein the change is at least about -2%, -3%, -4%, -5%, -6%, -7%, -8% or -10%. In further embodiments, a method is provided for increasing FVCpp in a subject with MD by administering an effective amount of an anti-CTGF antibody. In additional embodiments, treatment with an effective amount of an anti-CTGF antibody increases FVCpp by at least 0.5%, 1%, 1.5%, 2.0%, 2.5%, 3.0%, 4.0%, 5.0%, 6.0%, 7.0%, 8.0%, 9.0%, 10%, 15%, 20%, 30%, 40% or 50% compared to a subject's baseline FVCpp or to a historic control(s).

In some embodiments, the pathological change in pulmonary function is a change in forced expiratory volume (FEV), in particular FEV at 1 second (FEV1). In further embodiments, a method is provided for increasing FEV in a subject with MD by administering an effective amount of an anti-CTGF antibody. In other embodiments, the pathological change in pulmonary function is a change in PEF or PEFRpp. In additional embodiments, a method is provided for increasing PEF or PEFRpp in a subject with MD by administering an effective amount of an anti-CTGF antibody.

In further embodiments, treatment with the anti-CTGF antibody is for at least 3 weeks, 6 weeks, 9 weeks, 12 weeks, 15 weeks, 18 weeks, 21 weeks, 24 weeks, 27 weeks, 30 weeks, 33 weeks, 36 weeks or 48 weeks. In other embodiments, treatment is for 3 weeks or less, 6 weeks or less, 9 weeks or less, 12 weeks or less, 18 weeks or less, 24 weeks or less, 36 weeks or less, 48 weeks or less, 12 months or less, 16 months or less, 20 months or less, or 24 months or less from starting treatment with an anti-CTGF antibody. For example, if a subject with DMD has a baseline FVCpp of 65%, treatment with an anti-CTGF antibody raises the subject's FVCpp to 66.5% at week 36 post-initiation of therapy.

In one aspect, the invention provides a method of treating pulmonary dysfunction in a subject with a muscular dystrophy, the method comprising administering to a subject in need thereof an effective amount of an anti-CTGF agent, thereby treating the subject's pulmonary dysfunction. In some embodiments, the invention provides a method of improving pulmonary function in a subject with a muscular dystrophy, the method comprising administering to a subject in need thereof an effective amount of an anti-CTGF agent, thereby improving the subject's pulmonary function. The improvement in pulmonary function includes a reduction in the rate of decline of a parameter or specific measurement of pulmonary function as compared to the expected rate of decline as would be seen in a matched control group or a historical control(s). The improvement in pulmonary function further includes the stabilization of a pulmonary function measurement, i.e., a substantially similar reading is obtained at a later time point as compared to a baseline measurement, in contrast to an expected decline in that pulmonary function measurement that would be seen with a matched control group or a historical control(s). In particular embodiments, the comparison measurement is taken at about 1, 2, 3, 4, 5, 6, 7, 8, 9, 10, 11, 12, 16, 18, 20 or 24 months from the initiation of treatment and can be expressed as an annualized rate of change.

In some embodiments, the improvement in pulmonary function is a stabilization of FVC measured over time, i.e., no further loss of FVC during the time interval. In other embodiments, the improvement in pulmonary function is an increase in FVC. In particular embodiments, treatment with an effective amount of an anti-CTGF antibody increases FVC , at 1-year post treatment initiation, by at least 0.05 liters, 0.1 liters, 0.15 liters, 0.20 liters, 0.25 liters or 0.3 liters compared to a subject's baseline FVC or to historic controls.

In some embodiments, the improvement in pulmonary function is a reduction in the expected FVCpp compared to the FVCpp of a matched control or a historical control(s). In specific embodiments, the change from baseline in FVCpp at 1-year is less than about -1.0%, -2.0%, -3.0%, -4.0% or -5.0%. In other embodiments, the improvement of pulmonary function is the stabilization of FVCpp, i.e., no further decline in FVCpp over the time interval. In other embodiments, the improvement in pulmonary function is an increase in FVCpp. In particular embodiments, treatment with an effective amount of an anti-CTGF antibody increases FVCpp , at 1-year post treatment initiation, by at least 1%, 2.0%, 3.0%, 4.0%, 5.0%, 6.0%, 7.0%, 8.0%, 9.0%, or 10%.

In some embodiments, the improvement in pulmonary function is a stabilization of FEV, in particular, FEV at 1 second (FEV1), i.e., no further loss of FEV or FEV1 over the time interval. In other embodiments, the improvement in pulmonary function is an increase in FEV, in particular FEV1. In specific embodiments, treatment with an effective amount of an anti-CTGF antibody increases FEV, including FEV1, at 1-year post treatment initiation, by at least 1%, 2.0%, 3.0%, 4.0%, 5.0%, 6.0%, 7.0%, 8.0%, 9.0%, 10%, 15%, 20%, 30%, 40% or 50%.

In some embodiments, the improvement in pulmonary function is a reduction in the expected FEV% predicted (FEVpp) and in particular, FEV1% predicted (FEV1%pp). In specific embodiments, treatment with an effective amount of an anti-CTGF antibody results in a FEV1pp, at 1-year post treatment initiation, of less than about -1.0%, -2.0%, -3.0%, -4.0%, - 5.0%, -6.0%, -7.0%, -8.0%, -9.0% or -10.0%. In other embodiments, the improvement of pulmonary function is the stabilization of FEV1pp, i.e., no further decline in FEVlpp over the time interval. In further embodiments, the improvement in pulmonary function is an increase in FEV1pp. In particular embodiments, treatment with an effective amount of an anti-CTGF antibody increases FEV1pp, at 1-year post treatment initiation, by at least 1%, 2.0%, 3.0%, 4.0%, 5.0%, 6.0%, 7.0%, 8.0%, 9.0%, or 10%.

In further embodiments, the improvement in pulmonary function is a reduction in the expected rate of decline in PEF or peak expiratory flow rate % predicted (PEFRpp). In specific embodiments, treatment with an effective amount of an anti-CTGF antibody reduces the expected rate of decline in PEFRpp, at 1-year post treatment initiation, at least -1.0%, -2.0, - 3.0%, or -4.0%. In other embodiments, the improvement in pulmonary function is a stabilization of PEF or PEFRpp, i.e., no further decline in PEF or PEFRpp over the time interval. In further embodiments, the improvement in pulmonary function is an increase in PEF or PEFRpp over the time interval. In particular embodiments, treatment with an effective amount of an anti-CTGF antibody increases, at 1-year post treatment initiation, PEFRpp by at least 1%, 2.0%, 3.0%, 4.0%, 5.0%, 6.0%, 7.0%, 8.0%, 9.0%, or 10%.

Almost all DMD patients who survive to the third decade of life display cardiomyopathy. Recognition may be delayed by relative physical inactivity obscuring symptomatology. Typically seen are increased R/S ratio in the right precordial ECG leads, deep Q waves in the lateral leads, conduction abnormalities and arrhythmias (mainly supraventricular but also ventricular). Becker muscular dystrophy patients, whose skeletal myopathy occurs later and progresses more slowly, experience worse cardiomyopathy than DMD patients: up to 70% have LV dysfunction by echocardiography. The pathology of cardiomyopathy in patients with DMD and BMD classically produces subepicardial fibrosis of the inferolateral wall. Cardiac involvement in Emery-Dreifuss muscular dystrophy patients is common and usually becomes evident in the third decade as muscle weakness progresses, though cardiac manifestations have also been reported in young adults without muscle weakness. In EDMD, normal myocardium is gradually replaced by fibrous and adipose tissue, a process that usually starts in the atria (leading to atrial arrhythmias), often involves the atrioventricular node (leading to conduction abnormalities sometimes requiring pacemaker implantation) and eventually affects the ventricles (causing progressive dilatation and systolic failure). Cardiomyopathy is usually diagnosed and monitored in muscular dystrophy patients by magnetic resonance imaging (MRI), typically by evaluating the change in shortening fraction or ejection fraction over time, however, other techniques can be used including electrocardiography and echocardiography. A more sensitive MRI technique was recently developed, the serial analysis of left ventricular myocardial peak circumferential strain ($\varepsilon_{cc}$). This technique is able to detect myocardial strain abnormalities in DMD patients earlier than the standard MRI techniques (Hagenbuch et al. *supra;* Hor et al. J AM Coll Cardiol. 2009;April 7:53(14):1204-1210).

In one aspect, the invention provides a method of treating cardiac dysfunction in a subject with a muscular dystrophy, the method comprising administering to a subject in need thereof an effective amount of an anti-CTGF agent, thereby treating the subject's cardiac dysfunction. In another aspect, the invention provides a method of treating cardiac function in a subject with a muscular dystrophy, the method comprising administering to a subject in need thereof an effective amount of an agent that inhibits connective tissue growth factor (CTGF), thereby treating the subject's cardiac function. In another aspect, the invention provides a method of treating cardiomyopathy in a subject having a muscular dystrophy, the method comprising administering an effective amount of an anti-CTGF antibody. In further embodiments, the invention provides a method of improving cardiac function in a subject with a muscular dystrophy, the method comprising administering to a subject in need thereof an effective amount of an anti-CTGF agent, thereby improving the subject's cardiac function. In other embodiments, the invention provides a method of reducing the development of or reducing the progression rate of cardiac fibrosis in a subject with a muscular dystrophy, the method comprising administering to a subject in need thereof an effective amount of an anti-CTGF agent, thereby reducing the development of or reducing the progression rate of cardiac fibrosis. The improvement in cardiac function includes a reduction in the rate of decline of a parameter or specific measurement of cardiac function as compared to the expected rate of decline as would be seen in a matched control group or a historical control(s). The improvement in cardiac function further includes the stabilization of a cardiac function measurement, i.e., a substantially similar reading is obtained at a later time point as compared to a baseline measurement, in contrast to an expected decline in that cardiac function measurement that would be seen with a matched control group or a historical control(s). In particular embodiments, the comparison measurement is taken at about 1, 2, 3, 4, 5, 6, 7, 8, 9, 10, 11, 12, 16, 18, 20 or 24 months from the initiation of treatment and can be expressed as an annualized rate of change.

In some embodiments, administering an effective amount of an anti-CTGF antibody reduces the decline in LVEF%, stabilizes LVEF% or improves LVEF% in a MD subject. In other embodiments, administering an effective amount of an anti-CTGF antibody reduces the decline, stabilizes, or improves circumferential peak strain or global circumferential strain in a subject with a MD. In further embodiments, the method reduces the development of cardiac fibrosis, arrests the development of cardiac fibrosis or decreases the extent of cardiac fibrosis in a MD subject.

In some embodiments, the improvement in cardiac function is a change in LVEF%, circumferential peak strain or global circumferential strain compared to a subject's baseline measurement or compared to a matched control, wherein the change is at least about 1%, 2%, 3%, 4%, 5%, 6%, 7%, 8% or 10% greater than the baseline measurement or matched control. In some embodiments, the baseline measurement is taken, for example, 4 weeks, 8 weeks, 12 weeks, 24 weeks, 36 weeks, 48 weeks, or 12 months prior to the subsequent measurement.

In some embodiments, the improvement in cardiac function is an improvement in LVEF or LVEF%. In specific embodiments, treatment with an effective amount of an anti-CTGF antibody reduces the decline in LVEF or LVEF% when compared to a baseline measurement, a control group or a historical control(s). In other embodiments, treatment with an effective amount of an anti-CTGF antibody stabilizes or improves LVEF or LVEF%. In specific embodiments, treatment with an anti-CTGF antibody improves LVEF, at 1-year post treatment initiation, by at least 0.1%, 0.25%, 0.5%, 1%, 2%, 3%, 4%, 5%, 6%, 7%, 8%, 9%, 10%, 15% or 20%. In other embodiments, administering an effective amount of an anti-CTGF antibody reduces the decline, stabilizes, or improves circumferential peak strain or global circumferential strain in a subject with a MD. In specific embodiments, treatment with an anti-CTGF antibody improves circumferential peak strain or global circumferential strain, at 1-year post treatment initiation, by at least 0.1%, 0.25%, 0.5%, 1%, 2%, 3%, 4%, 5%, 6%, 7%, 8%, 9%, 10%, 15% or 20%.

In some embodiments, the method reduces the development of cardiac fibrosis, arrests the development of cardiac fibrosis (*e.g.* stabilizes i.e., no further development) or decreases the extent of cardiac fibrosis in a MD subject. In specific embodiments, treatment with an effective amount of anti-CTGF antibody reduces cardiac fibrosis, at 1-year post treatment initiation, by at least 0.1%, 0.25%, 0.5%, 1%, 2%, 3%, 4%, 5%, 6%, 7%, 8%, 9%, 10%, 15%, 20%, 25%, 30% or 40%. In particular embodiments, the change in cardiac fibrosis at 1-year, as assessed by mass late gadolinium enhancement (MLGE), is at least -0.1 g, -0.25 g, -0.5 g, -0.75 g, -1.0 g, -1.25 g, - 1.5 g or -2.0 g.

In some embodiments, the improvement in cardiac function is a change in LVEF%, circumferential peak strain or global circumferential strain compared to a subject's baseline measurement or compared to a matched control, wherein the change is at least about 1%, 2%, 3%, 4%, 5%, 6%, 7%, 8% or 10% greater than the baseline measurement or matched control. In some embodiments, the baseline measurement is taken, for example, 4 weeks, 8 weeks, 12 weeks, 24 weeks, 36 weeks, 48 weeks, or 12 months prior to the subsequent measurement.

Muscular strength can be assessed in ambulatory subjects using the North Star Ambulatory Assessment (NSAA) and the 6 min walking test (6MWT). Maintaining upper limb strength in non-ambulatory DMD patients is very important because it provides for functional independence. Upper limb strength in non-ambulatory subjects can be assessed using various evaluation systems known in the arts, including the Jebsen test of hand function, the Brooke score, the Egen Klassification scale, the Hammersmith Motor Ability Score and the motor function measure score.

In one aspect, the invention provides for a method for increasing muscle strength, the method comprising administering an effective amount of an anti-CTGF agent, such as an anti-CTGF antibody. In further embodiments, the method reduces the decline in muscle strength or arrests (*e.g.* stabilizes) the decline in muscle strength or increases muscle strength in a MD subject. In specific embodiments, treatment with an anti-CTGF antibody increases hand grip strength, when measured at 1-year post treatment initiation, by at least 0.5, 1.0, 2.0, 3.0, 4.0, 5.0, 6.0, 7.0, 8.0, 9.0 or 10.0 newton. In particular embodiments, treatment with an anti-CTGF antibody stabilizes (no further decline) or increases a PUL assessment score when measured at 1-year post treatment initiation. In further embodiments, the increase in PUL assessment score is at least 1 unit.

In other embodiments, a method is provided for reducing muscle inflammation, muscle edema, fat infiltration or reduce the percentage of dystrophic muscle in a subject with a muscular dystrophy, comprising administering an effective amount of an anti-CTGF antibody. A further means to assess disease progression and response to therapy is to monitor muscle inflammation, muscle edema, dystrophic muscle composition and fat infiltration over time. The degree of muscle inflammation, muscle edema, fat infiltration or percentage of dystrophic muscle can be readily obtained using diagnostic imaging technology including magnetic resonance imaging (MRI), ultrasound and computed tomography (CT). With MRI, typically, edema can be identified using T2 or short-time inversion recovery (STIR) sequences, while fatty infiltration can be identified using T1 or 3-point Dixon sequences (Diaz-Manera et al. Acta Myolgica. 2015; 34:95-108). In particular embodiments, administering an effective amount of an anti-CTGF antibody reduces, at 1-year post treatment initiation, muscle inflammation, muscle edema, dystrophic muscle composition and fat infiltration by at least 0.1%, 0.2%, 0.3%, 0.4%, 0.5%, 0.6%, 0.7%, 0.8%, 0.9%, 1.0%, 1.5%, 2.0%, 3.0%, 4.0%, 5.0%, 6.0%, 7.0%, 8.0%, 9.0% or 10%.

**Subjects**

In some embodiments, the subjects suitable for, or in need of treatment of the methods and anti-CTGF agents of the present invention are mammals, more preferably humans, who are at risk of developing a MD or have already displayed at least one symptom of a MD. Early symptoms of MDs often seen at the time of clinical presentation include waddling gait, walking on toes, trouble running and jumping, frequent falls, large calf muscles, muscle pain and stiffness, difficulty rising from lying or sitting position. Later appearing symptoms of a MD include loss of ambulation, respiratory problems, contractures, scoliosis, and cardiomyopathy.

Subjects suspected of having a MD can be readily identified by any competent medical practitioner using standard diagnostic tests and criteria including blood tests for serum creating phosphokinase, electromyography, and muscle biopsies.

Genetic testing may also be employed to diagnose a subject with a MD. Techniques used in genetic testing include the polymerase chain reaction (PCR), Southern blotting, mutation scanning, and/or sequence analysis. DNA can be extracted from any relevant tissue or cell type including muscle cells. The identification and treatment of subjects with a MD, including DMD, or with a propensity to develop a MD, using genetic testing is specifically contemplated. Treatment of subjects with an anti-CTGF agent before the development of overt clinical symptoms of a MD may delay or prevent the development of clinical symptoms, thereby increasing the life span or quality of life of an affected individual.

## Agents

In any of the methods described above, it is particularly contemplated that the agent or medicament that inhibits CTGF (i.e., the anti-CTGF agent or medicament) may be a polypeptide, polynucleotide, or small molecule; for example, an antibody that binds to CTGF, a CTGF antisense molecule, miRNA, ribozyme or siRNA, a small molecule chemical compound, etc. In some embodiments, inhibition of CTGF is accomplished using an antibody that specifically binds CTGF. In further embodiments, the invention contemplates inhibiting CTGF using an anti-CTGF oligonucleotide, but inhibition of CTGF can be accomplished by any of the means well-known in the art for modulating the expression or activity of CTGF.

## Antibodies

The term "antibody" is used in the broadest sense and specifically covers monoclonal antibodies (including full length monoclonal antibodies), polyclonal antibodies, multispecific antibodies (e.g., bispecific antibodies), and antibody fragments, so long as they exhibit the desired biological activity. The term antibody further includes antibody mimetics, discussed further below.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo,* to create a multispecific antibody, etc., and that an antibody comprising the altered target binding sequence is also a monoclonal antibody of this invention. In contrast to polyclonal antibody preparations, which typically include different antibodies directed against different determinants (epitopes), each monoclonal antibody of a monoclonal antibody preparation is directed against a single determinant on an antigen.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler G and Milstein C, Nature, 256:495-497 (1975); Harlow E et al., Antibodies: A Laboratory Manual (Cold Spring Harbor Laboratory Press, 2nd ed. (1988); recombinant DNA methods (see, e.g., U.S. Pat. No. 4,816,567); phage-display technologies (see, e.g., Clackson T et al., Nature, 352: 624-628 (1991); Marks JD et al., J Mol Biol 222: 581-597 (1992); and Lee V et al., J Immunol Methods 284(1-2): 119-132 (2004)), and technologies for producing human or human-like antibodies in animals that have parts or all of the human immunoglobulin loci or genes encoding human immunoglobulin sequences (see, e.g., WO 1998/24893; WO 1996/34096; WO 1996/33735; WO 1991/10741; Jakobovits A et al., Proc Natl Acad Sci USA 90: 2551 (1993); U.S. Pat. Nos. 5,545,807; 5,545,806; 5,569,825; 5,625,126; 5,633,425; and 5,661,016).

Monoclonal antibodies specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass (see, e.g., U.S. Pat. No. 4,816,567; and Morrison S et al., Proc Natl Acad Sci USA 81:6851-6855 (1984)).

"Humanized" forms of non-human (e.g., murine) antibodies are chimeric antibodies that contain minimal sequence derived from non-human immunoglobulin. In some embodiments, a humanized antibody is a human immunoglobulin (recipient antibody) in which residues from a one or more hypervariable regions (HVRs) of the recipient are replaced by residues from one or more HVRs of a non-human species (donor antibody) such as mouse, rat, rabbit, or nonhuman primate having the desired specificity, affinity, and/or capacity. For further details, see, e.g., Jones TA et al., Nature 321:522-525 (1986); Riechmann L et al., Nature 332:323-329 (1988); and U.S. Pat. Nos. 6,982,321 and 7,087,409.

A "human antibody" is one which possesses an amino acid sequence which corresponds to that of an antibody produced by a human and/or has been made using any of the techniques for making human antibodies (see e.g., Hoogenboom HR and Winter G, J. Mol. Biol., 227:381 (1992); Marks JD et al., J. Mol. Biol., 222:581 (1991); Boerner R et al., J. Immunol., 147(1):86-95 (1991); Li J et al., Proc. Natl. Acad. Sci. USA, 103:3557-3562 (2006) and U.S. Pat. Nos. 6,075,181 and 6,150,584).

The anti-CTGF antibodies of the invention may be specific for CTGF endogenous to the species of the subject to be treated or may be cross-reactive with CTGF from one or more other species. In some embodiments, the antibody for use in the present methods is obtained from the same species as the subject in need. In other embodiments, the antibody is a chimeric antibody wherein the constant domains are obtained from the same species as the subject in need and the variable domains are obtained from another species. For example, in treating a human subject the antibody for use in the present methods may be a chimeric antibody having constant domains that are human in origin and variable domains that are mouse in origin. In preferred embodiments, the antibody for use in the present methods binds specifically to the CTGF endogenous to the species of the subject in need. Thus, in certain embodiments, the antibody is a human or humanized antibody, particularly a monoclonal antibody, that specifically binds human CTGF (GenBank Accession No. NP_001892).

Exemplary antibodies for use in the treatment methods of the present invention are described, e.g., in U.S. Patent No. 5,408,040; PCT/US1998/016423; PCT/US1999/029652; International Publication No. WO 99/33878; US Pat No. 9587015; WO 2013/108869 and Myzithras et al. Bioanalysis. 2018 Mar 1;10(6):397-406. Preferably, the anti-CTGF antibody is a monoclonal antibody. Preferably the antibody is a neutralizing antibody. In particular embodiments, the antibody is the antibody described and claimed in United States Patent Nos. 7,405,274 and 7,871,617. In some embodiments, the antibody for treatment of MDs has the amino acid sequence of the antibody produced by the cell line identified by ATCC Accession No. PTA-6006. In other embodiments, the antibody binds to CTGF competitively with an antibody produced by ATCC Accession No. PTA-6006. In other embodiments, the anti-CTGF antibody binds to domain 2 of human CTGF. In further embodiments, the antibody binds to the same epitope as the antibody produced by ATCC Accession No. PTA-6006. A particular antibody for use in the disclosed treatment methods is CLN1 or mAb1 as described in U.S. Patent No. 7,405,274, or an antibody substantially equivalent thereto or derived therefrom. In some embodiments, the anti-CTGF antibody is CLN1, an antibody identical to the antibody produced by the cell line identified by ATCC Accession No. PTA-6006 that is encompassed by the claims of United States Patent Nos. 7,405,274 and 7,871,617. In specific embodiments, the anti-CTGF antibody is pamrevlumab (CAS# 946415-13-0).

As referred to herein, the phrase "an antibody that specifically binds to CTGF" includes any antibody that binds to CTGF with high affinity. Affinity can be calculated from the following equation:

$$Affinity = K_a = \frac{[Ab \cdot Ag]}{[Ab][Ag]} = \frac{1}{K_d}$$

where [Ab] is the concentration of the free antigen binding site on the antibody, [Ag] is the concentration of the free antigen, [Ab· Ag] is the concentration of occupied antigen binding sites, $K_a$ is the association constant of the complex of antigen with antigen binding site, and $K_d$ is the dissociation constant of the complex. A high-affinity antibody typically has an affinity at least on the order of $10^8$ $M^{-1}$, $10^9$ $M^{-1}$ or $10^{10}$ $M^{-1}$. In particular embodiments, an antibody for use in the present methods will have a binding affinity for CTGF between of $10^8$ $M^{-1}$ and $10^{10}$ $M^{-1}$, between $10^8$ $M^{-1}$ and $10^9$ $M^{-1}$ or between $10^9$ $M^{-1}$ and $10^{10}$ $M^{-1}$. In some embodiments the high-affinity antibody has an affinity of about $10^8$ $M^{-1}$, $10^9$ $M^{-1}$ or $10^{10}$ $M^{-1}$.

"Antibody fragments" comprise a functional fragment or portion of an intact antibody, preferably comprising an antigen binding region thereof. A functional fragment of an antibody will be a fragment with similar (not necessarily identical) specificity and affinity to the antibody which it is derived. Non-limiting examples of antibody fragments include Fab, $F(ab')_2$, and Fv fragments that can be produced through enzymatic digestion of whole antibodies, e.g., digestion with papain, to produce Fab fragments. Other non-limiting examples include engineered antibody fragments such as diabodies (Holliger P et al. Proc Natl Acad Sci USA, 90: 6444-6448 (1993)); linear antibodies (Zapata G et al. Protein Eng, 8(10):1057-1062 (1995)); single-chain antibody molecules (Bird KD et al. Science, 242: 423-426 (1988)); single domain antibodies, also known as nanobodies (Ghahoudi MA et al. FEBS Lett. 414: 521-526, (1997)); domain antibodies (Ward ES et al. Nature. 341: 544-546, (1989)); and multispecific antibodies formed from antibody fragments.

## Antibody Mimetics

Antibody mimetics are proteins, typically in the range of 3-25 kD, that are designed to bind an antigen with high specificity and affinity like an antibody, but are structurally unrelated to antibodies. Frequently, antibody mimetics are based on a structural motif or scaffold that can be found as a single or repeated domain from a larger biomolecule. Examples of domain-derived antibody mimetics include AdNectins that utilize the 10th fibronectin III domain (Lipovšek D. Protein Eng Des Sel, 24:3-9 (2010)); Affibodies that utilize the Z domain of *staphylococcal* protein A (Nord K et al. Nat Biotechnol. 15: 772-777 (1997)), and DARPins that utilize the consensus ankyrin repeat domain (Amstutz P. Protein Eng Des Sel. 19:219-229 (2006)). Alternatively, antibody mimetics can also be based on the entire structure of a smaller biomolecule, such as Anticalins that utilize the lipocalin structure (Beste G et al. Proc Natl Acad Sci USA. 5:1898-1903 (1999)). In some embodiments, the anti-CTGF antibody is an antibody mimetic.

**Oligonucleotides**

In some aspects, the present invention comprises synthetic oligonucleotides that decrease the expression of human CTGF mRNA. These anti-CTGF oligonucleotides include isolated nucleic acids, nucleic acid mimetics, and combinations thereof. Oligonucleotides of the invention comprise antisense oligonucleotides, ribozymes, external guide sequence (EGS) oligonucleotides (oligozymes) and inhibitory RNA (RNAi) including siRNA, microRNA (miRNA), and short hairpin RNA (shRNA). Oligonucleotides that decrease the expression of CTGF mRNA are useful for treating MDs and in particular, DMD.

The terms "oligonucleotide" and "oligomeric nucleic acid" refer to oligomers or polymers of ribonucleic acid (RNA), deoxyribonucleic acid (DNA), mimetics or analogs of RNA or DNA, or combinations thereof, in either single- or double-stranded form. Oligonucleotides are molecules formed by the covalent linkage of two or more nucleotides or their analogs.

The terms "complementary" and "complementarity" refer to conventional Watson-Crick base-pairing of nucleic acids. For example, in DNA complementarity, guanine forms a base pair with cytosine and adenine forms a base pair with thymine, whereas in RNA complementarity, guanine forms a base pair with cytosine, but adenine forms a base pair with uracil in place of thymine. An oligonucleotide is complementary to a RNA or DNA sequence when the nucleotides of the oligonucleotide are capable of forming hydrogen bonds with a sufficient number of nucleotides in the corresponding RNA or DNA sequence to allow the oligonucleotide to hybridize with the RNA or DNA sequence. In some embodiments, the oligonucleotides have perfect complementarity to human CTGF mRNA, i.e., no mismatches.

When used in the context of an oligonucleotide, "modified" or "modification" refers to an oligonucleotide that incorporates one or more unnatural (modified) sugar, nucleobase or internucleoside linkage. Modified oligonucleotides are structurally distinguishable, but functionally interchangeable with naturally occurring or synthetic unmodified oligonucleotides and usually have enhanced properties such as increased resistance to degradation by exonucleases and endonucleases, or increased binding affinity. In some embodiments, the anti-CTGF oligonucleotides are modified.

Unnatural covalent internucleoside linkages, i.e., modified backbones, include those linkages that retain a phosphorus atom in the backbone and also those that do not have a phosphorus atom in the backbone. Numerous phosphorous containing modified oligonucleotide backbones are known in the art and include, for example, phosphoramidites, phosphorodiamidate morpholinos, phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, and phosphinates. See Swayze E and Bhat B in Antisense Drug Technology Principles, Strategies, and Applications. 2nd Ed. CRC Press, Boca Rotan FL, p. 144-182 (2008).

In further embodiments, the unnatural internucleoside linkages are uncharged and in others, the linkages are achiral. In some embodiments, the unnatural internucleoside linkages are uncharged and achiral, e.g., peptide nucleic acids (PNAs).

In some embodiments, the modified sugar moiety is a sugar other than ribose or deoxyribose. In certain embodiments, the sugar is arabinose, xylulose or hexose. In further embodiments, the sugar is substituted with one of the following at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C1 to C10 alkyl or C2 to C10 alkenyl and alkynyl. In some embodiments, the modifications include 2'-methoxy (2'-O--CH3), 2'-aminopropoxy (2'-OCH2CH2CH2NH2), 2'-allyl (2'-CH2-CH=CH2), 2'-O-allyl (2'-O--CH2-CH=CH2) and 2'-fluoro (2'-F). The 2'-modification may be in the arabino (up) position or ribo (down) position. Similar modifications may also be made at other positions on an oligonucleotide, particularly the 3' position of the sugar on the 3' terminal nucleotide or in 2'-5' linked oligonucleotides and the 5' position of 5' terminal nucleotide.

In some embodiments, the modified sugar is conformationally restricted. In further embodiments, the conformational restriction is the result of the sugar possessing a bicyclic moiety. In other embodiments, the bicyclic moiety links the 2'-oxygen and the 3' or 4'-carbon atoms. In additional embodiments the linkage is a methylene (--CH2--)n group bridging the 2' oxygen atom and the 4' carbon atom, wherein n is 1 or 2. This type of structural arrangement produces what are known as "locked nucleic acids" (LNAs). See Koshkin AA et al. Tetrahedron, 54, 3607-3630 (1998); and Singh SK et al., Chem. Commun, 4:455-456 (1998).

In some embodiments, the sugar is a sugar mimetic that is conformationally restricted resulting in a conformationally constrained monomer. In certain embodiments, the sugar mimetic comprises a cyclohexyl ring that comprises one ring heteroatom and a bridge making the ring system bicyclic. See PCT/US2010/044549. In further embodiments, the oligonucleotides comprise at least one nucleotide that has a bicyclic sugar moiety or is otherwise conformationally restricted.

In some embodiments, the modified sugar moiety is a sugar mimetic that comprises a morpholino ring. In further embodiments, the phosphodiester internucleoside linkage is replaced with an uncharged phosphorodiamidate linkage. See Summerton J and Weller D, Antisense Nucleic Acid Drug Dev, 7:187 -195 (1997).

In some embodiments, both the phosphate groups and the sugar moieties are replaced with a polyamide backbone comprised of repeating N-(2-aminoethyl)-glycine units to which the nucleobases are attached via methylene carbonyl linkers. These constructs are called peptide nucleic acids (PNAs). PNAs are achiral, uncharged and because of the peptide bonds, resistant to endo- and exonucleases. See Nielsen PE et al., Science, 254:1497-1500 (1991) and U.S. Pat. No. 5,539,082.

Oligonucleotides useful in the methods of the invention include those comprising entirely or partially of naturally occurring nucleobases. Naturally occurring nucleobases include adenine, guanine, thymine, cytosine, uracil, 5-methylcytidine, pseudouridine, dihydrouridine, inosine, ribothymidine, 7-methylguanosine, hypoxanthine and xanthine.

Oligonucleotides further include those comprising entirely or partially of modified nucleobases (semisynthetically or synthetically derived). See Herdewijn P, Antisense Nucleic Acid Drug Dev 10: 297-310 (2000); and Sanghvi Y S, et al. Nucleic Acids Res, 21: 3197-3203 (1993).

In some embodiments, at least one nucleoside, i.e., a joined base and sugar, in an oligonucleotide is modified, i.e., a nucleoside mimetic. In certain embodiments, the modified nucleoside comprises a tetrahydropyran nucleoside, wherein a substituted tetrahydropyran ring replaces the naturally occurring pentofuranose ring. See PCT/US2010/022759 and PCT/US2010/023397. In other embodiments, the nucleoside mimetic comprises a 5'-substituent and a 2'-substituent. See PCT/US2009/061913. In some embodiments, the nucleoside mimetic is a substituted $\alpha$-L-bicyclic nucleoside. See PCT/US2009/058013. In additional embodiments, the nucleoside mimetic comprises a bicyclic sugar moiety. See PCT/US2009/039557. In further embodiments, the nucleoside mimetic comprises a bis modified bicyclic nucleoside. See PCT/US2009/066863. In certain embodiments, the nucleoside mimetic comprises a bicyclic cyclohexyl ring wherein one of the ring carbons is replaced with a heteroatom. See PCT/US2009/033373. In still further embodiments, a 3' or 5'-terminal bicyclic nucleoside is attached covalently by a neutral internucleoside linkage to the oligonucleotide. See PCT/US2009/039438. In other embodiments, the nucleoside mimetic is a tricyclic nucleoside. See PCT/US2009/037686.

The oligonucleotides of the invention can contain any number of the modifications described herein. The aforementioned modifications may be incorporated uniformly across an entire oligonucleotide, at specific regions or discrete locations within the oligonucleotide including at a single nucleotide. Incorporating these modifications can create chimeric or hybrid oligonucleotides wherein two or more chemically distinct areas exist, each made up of one or more nucleotides.

Oligonucleotides of the invention can be synthesized by any method known in the art, e.g., using enzymatic synthesis and/or chemical synthesis. The oligonucleotides can be synthesized in vitro (e.g., using enzymatic synthesis and chemical synthesis) or in vivo (using recombinant DNA technology well known in the art). In a preferred embodiment, chemical synthesis is used for modified polynucleotides. Chemical synthesis of linear oligonucleotides is well known in the art and can be achieved by solution or solid phase techniques. Preferably, synthesis is by solid phase methods. Automated, solid phase oligonucleotide synthesizers used to construct the oligonucleotides of the invention are available through various vendors including GE Healthcare Biosciences (Piscataway, NJ).

Oligonucleotide synthesis protocols are well known in the art and can be found, e.g., in U.S. Pat. No. 5,830,653; WO 98/13526; Stec W et al. JAm Chem Soc 106:6077-6079 (1984); Stec W et al. J Org. Chem 50:3908-3913 (1985); Stec W et al. J Chromatog 326:263-280 (1985); LaPlanche JL et al. Nucl Acid Res 26:251-60 (1986); Fasman G D, Practical Handbook of Biochemistry and Molecular Biology (1989). CRC Press, Boca Raton, Fla.; U.S. Pat. No. 5,013,830; U.S. Pat. No. 5,214,135; U.S. Pat. No. 5,525,719; WO 92/03568; U.S. Pat. No. 5,276,019; and U.S. Pat. No. 5,264,423.

As used herein, the term "antisense oligonucleotide" refers to an oligomeric nucleic acid that is capable of hybridizing with its complementary target nucleic acid sequence resulting in the impairment of the normal function of the target nucleic acid sequence. Antisense oligonucleotides that inhibit CTGF expression have been described and utilized to decrease CTGF expression in various cell types. (See, e.g., PCT/US 1996/008140; PCT/US 1999/026189; PCT/US1999/029652; PCT/US2002/038618; Kothapalli D et al. Cell Growth Differ 8:61-68, 1997; Shimo T et al. J Biochem (Tokyo) 124:130-140 (1998); Uchio K et al. Wound Repair Regen 12:60-66 (2004); Guha M et al. FASEB J 21:3355-3368 (2007); U.S. Patent No. 6,358,741; U.S. Patent No. 6,965,025; U.S. Patent No. 7,462,602; U.S. Patent Application Publication No. 2008/0070856; U.S. Patent Application Publication No. 2008/0176964; and U.S. Patent No. 8,802,839; PCT/US02/38618; PCT/US2009/054973; PCT/US2009/054974; PCT/US2009/054975; PCT/US2009/054976; PCT/US2012/023620; and U.S. Patent Application No. 13/364,547, incorporated herein by reference in their entirety.

In some embodiments, the oligonucleotides used to decrease the expression of human CTGF mRNA are small interfering RNA (siRNA). As used herein, the terms "small interfering RNA" or "siRNA" refer to single- or double-stranded RNA molecules that induce the RNA interference (RNAi) pathway and act in concert with host proteins, e.g., RNA induced silencing complex (RISC) to degrade mRNA in a sequence-specific fashion. In naturally occurring RNAi, a double-stranded RNA (dsRNA) is cleaved by the RNase III/helicase protein, Dicer, into small interfering RNA (siRNA) molecules. These siRNAs are incorporated into a multicomponent-ribonuclease called RNA-induced silencing complex (RISC). One strand of siRNA remains associated with RISC and guides the complex toward a cognate RNA that has sequence complementary to the guider ss-siRNA in RISC. This siRNA-directed endonuclease digests the RNA, thereby inactivating it.

Selective silencing of CTGF expression by RNAi can be achieved by administering isolated siRNA oligonucleotides or by the *in vivo* expression of engineered RNA precursors (see U.S. Patent Nos. 7,056,704, 7,078,196, 7,459,547, 7,691,995 and 7,691,997).

In some embodiments, treatment methods are provided wherein patients are administered a recombinant expression vector that expresses anti-CTGF oligonucleotides. Such genetic constructs can be designed using appropriate vectors and expressional regulators for cell- or tissue-specific expression and constitutive or inducible expression. These genetic constructs can be formulated and administered according to established procedures within the art. In some embodiments, patients are administered recombinant expression vectors that encode a short hairpin oligonucleotide. In further embodiments, the recombinant expression vectors are DNA plasmids, while in other embodiments, the expression vectors are viral vectors. RNA expressing viral vectors can be constructed based on, but not limited to, adeno-associated viruses, retroviruses, adenoviruses, or alphaviruses. In some embodiments, the expression vectors persist in target cells. Alternatively, such vectors can be repeatedly administered as necessary.

In some embodiments, the decrease in expression of CTGF mRNA by an anti-CTGF oligonucleotide comprises the interference in the function of the target CTGF DNA sequence (CTGF gene), typically resulting in decreased replication and/or transcription of the target CTGF DNA. In other embodiments, the decrease in expression of CTGF mRNA by an anti-CTGF oligonucleotide comprises the interference in function of CTGF RNA, typically resulting in impaired splicing of transcribed CTGF RNA (pre-mRNA) to yield mature mRNA species, decreased CTGF RNA stability, decreased translocation of the CTGF mRNA to the site of protein translation and impaired translation of protein from mature mRNA. In other embodiments, the decrease in expression of CTGF mRNA by an anti-CTGF oligonucleotide comprises the decrease in cellular CTGF mRNA number or cellular content of CTGF mRNA. In some embodiments, the decrease in expression of CTGF mRNA by an anti-CTGF oligonucleotide comprises the down-regulation or knockdown of CTGF gene expression. In other embodiments, the decrease in expression of CTGF mRNA by an anti-CTGF oligonucleotide comprises the decrease in CTGF protein expression or cellular CTGF protein content.

In some embodiments, the methods of the invention comprise the administration of an effective amount of an anti-CTGF oligonucleotide that decreases CTGF mRNA transcription rate, cellular CTGF mRNA level, CTGF expression rate, cellular CTGF protein level or interstitial CTGF protein level. In further embodiments, the methods of the invention comprise the administration of an effective amount of an anti-CTGF oligonucleotide that decreases CTGF mRNA transcription rate, cellular CTGF mRNA level, CTGF expression rate, cellular CTGF protein level by at least 5%, at least 10%, at least 15%, at least 20%, at least 25%, at least 30%, at least 35%, at least 40%, at least 45%, at least 50%, at least 60%, at least 70%, at least 80% or at least 90% compared to controls.

**Administration and Dosage**

An effective amount of an anti-CTGF agent or pharmaceutical composition thereof can be administered as often as necessary, e.g., once, twice or three times per day, every other day, once, twice or three times per week, every other week, every three weeks or monthly. The skilled artisan will appreciate that certain factors may influence the dosage and timing required to effectively treat a subject, including but not limited to the severity or extent of the disease, the administration route, previous treatments, concurrent medications, performance status, weight, gender, race or ethnicity, and/or age of the subject. In certain embodiments, the methods for treating a MD presented herein comprise the administration to a subject in need thereof an anti-CTGF agent at a range from about 0.01 mg to about 10,000 mg, from about 0.1 mg to about 5,000 mg, from about 1.0 mg to about 2,500 mg, from about 1.0 mg to about 1,000 mg, from about 10 mg to about 500 mg, from about 100 mg to about 1,000 mg, from about 0.10 mg to about 50 mg or from about 0.5 mg to about 50 mg.

In some embodiments, the methods for treating a MD presented herein comprise the administration to a subject in need thereof at least about 0.1 mg, 0.5 mg, 1.0 mg, 2.0 mg, 4 mg, 8 mg, 16 mg, 25 mg, 50 mg, 100 mg, 200 mg, 400 mg, 800 mg, 1,000 mg, 2,000 mg, 3,000 mg, 5,000 mg or 10,000 mg of an anti-CTGF agent. In some embodiments, the methods for treating a MD presented herein comprise the administration to a subject in need thereof not more than about 1 mg, 5 mg, 10 mg, 20 mg, 30 mg, 40 mg, 50 mg, 60 mg, 70 mg, 80 mg, 90 mg, 100 mg, 150 mg, 175 mg, 200 mg, 250 mg, 500 mg, 750 mg, 1,000, 2,000, 5,000 mg or 10,000 mg of an anti-CTGF agent.

In further embodiments, the methods for treating a MD presented herein comprise the administration to a subject in need thereof an anti-CTGF agent from about 0.001 mg/kg to about 5,000 mg/kg, about 0.01 mg/kg to about 1000 mg/kg, about 0.1 mg/kg to about 500 mg/kg or about 1.0 mg/kg to about 100 mg/kg.

In some embodiments, an effective amount of an anti-CTGF antibody is administered at a dose of between about 1 mg/kg to 100 mg/kg, 5 mg/kg to 75 mg/kg, 10 mg/kg to 50 mg/kg, 15 mg/kg to 45 mg/kg or 20 mg/kg to 45 mg/kg. In other embodiments, an effective amound of the anti-CTGF antibody comprises a dose of about 15 mg/kg, 20 mg/kg, 25 mg/kg, 30 mg/kg, 35 mg/kg, 40 mg/kg, 45 mg/kg or 50 mg/kg. In further embodiments, the anti-CTGF antibody is administered systemically, e.g., intravenous administration. In particular embodiments, the anti-CTGF antibody is administered at 35 mg/kg every two or three weeks. In further embodiments, the anti-CTGF antibody is pamrevlumab administered at 35 mg/kg every two or three weeks. In additional embodiments, treatment with the anti-CTGF antibody is for at least 3 weeks, 6 weeks, 9 weeks, 12 weeks, 15 weeks, 18 weeks, 21 weeks, 24 weeks, 27 weeks, 30 weeks, 33 weeks, 36 weeks or 48 weeks. In other embodiments, treatment is for 3 weeks or less, 6 weeks or less, 9 weeks or less, 12 weeks or less, 18 weeks or less, 24 weeks or less, 36 weeks or less, 48 weeks or less, 12 months or less, 16 months or less, 20 months or less, or 24 months or less.

In some embodiments, an effective amount of an anti-CTGF oligonucleotide comprises a dose between about 0.01 mg to about 1,000 mg, about 0.1 mg to about 100 mg, about 1.0 mg to about 50 mg, about 1.0 mg to about 25 mg, or about 5 mg to about 50 mg.

In further embodiments, the methods for treating a MD presented herein comprise the administration to a subject in need thereof of an effective amount of an anti-CTGF agent or a pharmaceutical composition thereof, at a dosage that achieves a target plasma, or tissue concentration of the anti-CTGF agent. In particular embodiments, the administered dosage achieves a plasma or tissue concentration of the anti-CTGF agent ranging from about 0.001 μg/mL to about 100 mg/mL, about 0.01 μg/mL to about 10 mg/mL, about 0.1 μg/mL to about 1 mg/mL or about 1 μg/mL to about 100 μg/ml in a subject with a MD. In other embodiments, the administration to a subject in need thereof of an anti-CTGF antibody achieves a plasma or tissue target concentration of the anti-CTGF antibody of at least about 10 μg/ml, at least about 50 μg/ml, at least about 100 μg/mL, at least about 200 μg/mL, at least about 300 μg/mL or at least about 400 μg/mL. In further embodiments, the administration to a subject in need thereof of an anti-CTGF antibody achieves a plasma or tissue target concentration of a range of about 1.0 μg/ml to about 2,000 μg/ml, about 10 μg/mL to about 1,000 μg/mL, or about 20 μg/mL to about 500 μg/mL.

In certain embodiments, subsequent doses of an anti-CTGF agent may be adjusted accordingly based on the plasma or tissue concentration of the anti-CTGF agent achieved with earlier doses of the anti-CTGF agent. In general, the dosage and frequency of administration of an anti-CTGF agent may be adjusted over time to provide sufficient levels of the anti-CTGF agent to maintain the desired effect.

## Combination Therapy

In some embodiments, the methods for treating a MD, e.g., DMD, provided herein involve the administration of an anti-CTGF agent in combination with one or more additional therapies. As used herein, the term "in combination" refers to the administration of the anti-CTGF agent prior to, concurrent with, or subsequent to the administration of one or more additional therapies for use in treating a MD or a symptom of a MD. The use of the term "in combination" does not restrict the order in which the anti-CTGF agent and the one or more additional therapies are administered to a subject. The additional therapies may be administered by the same route or a different route of administration than used for the anti-CTGF agent.

In some embodiments, the additional therapy administered in combination with the anti-CTGF agent is a drug or pharmaceutical composition comprising a drug. In particular embodiments, the anti-CTGF agent is administered in combination with a corticosteroid, e.g., prednisone, deflazacort or oxandrolone. In other embodiments, the anti-CTGF agent is administered in combination with ataluren or eteplirsen.

In further embodiments, the anti-CTGF agent is administered in combination with another therapy including exercise, ventilatory assistance (e.g., intermittent positive pressure ventilation, bilevel positive airway pressure (BiPAP), biphasic cuirass ventilation), occupational therapy, and/or physical therapy.

In specific embodiments, the interval of time between the administration of an anti-CTGF agent and the administration of one or more additional therapies may be about 0 to 15 minutes, 0 to 30 minutes, 30 minutes to 60 minutes, 1 to 2 hours, 2 to 6 hours, 2 to 12 hours, 12 to 24 hours, 1 to 2 days, 2 to 4 days, 4 to 7 days, 1 to 2 weeks, 2 to 4 weeks, 4 to 12 weeks, 12 to 24 weeks, or 24 to 52 weeks. In certain embodiments, an anti-CTGF agent and one or more additional therapies are administered less than 1 day, 1 week, 2 weeks, 3 weeks, 4 weeks, one month, 2 months, 3 months, 6 months, or 1 year apart.

In some embodiments, the administration of an anti-CTGF agent in combination with one or more additional therapies has an additive effect, while in other embodiments the combination of therapies have a synergistic effect. In specific embodiments, a synergistic effect achieved with combination therapy permits the use of lower dosages (e.g., sub-optimal conventional doses) of the additional therapy, e.g., a corticosteroid. In other embodiments, the synergistic effect achieved with combination therapy allows for a less frequent administration of the additional therapy to a subject. In certain embodiments, the ability to utilize lower dosages of an additional therapy and/or to administer the additional therapy less frequently reduces the toxicity associated with the administration of the additional therapy, without reducing the efficacy of the additional therapy. In some embodiments, a synergistic effect results in improved efficacy of an anti-CTGF antibody and/or the additional therapies in treating a MD.

The combination of an anti-CTGF agent and one or more additional therapies can be administered to a subject in the same pharmaceutical composition. Alternatively, an anti-CTGF agent and one or more additional therapies can be administered concurrently to a subject in separate pharmaceutical compositions. An anti-CTGF agent and one or more additional therapies may also be administered to a subject by the same or different routes of administration.

## Pharmaceutical Formulations and Routes of Administration

The compositions and compounds suitable for use in the methods of the present invention can be delivered directly or in pharmaceutical compositions containing excipients, as is well known in the art. Present methods of treatment comprise administration of an effective amount of an anti-CTGF agent to a subject having or at risk of developing a MD. An effective amount, e.g., dose, of compound or drug (e.g. an anti-CTGF agent), can readily be determined by routine experimentation, as can an effective and convenient route of administration and an appropriate formulation. Various formulations and drug delivery systems are available in the art and depend in part on the intended route of administration. (See, e.g., Gennaro AR, ed. Remington's Pharmaceutical Sciences, (2000); and Hardman JG, Limbird LE, and Gilman LS, eds. The Pharmacological Basis of Therapeutics, (2001)).

Suitable routes of administration may, for example, include oral, rectal, topical, nasal, pulmonary, intestinal, and parenteral administration. Primary routes for parenteral administration include intravenous, intramuscular, and subcutaneous administration. Secondary routes of administration include intraperitoneal and intraarterial administration.

Pharmaceutical dosage forms of an anti-CTGF agent for use in the invention may be provided in an instant release, controlled release, sustained release, or target drug-delivery system. Commonly used dosage forms include, for example, solutions and suspensions, (micro-) emulsions, ointments, gels and patches, liposomes, tablets, dragees, soft or hard shell capsules, suppositories, ovules, implants, amorphous or crystalline powders, aerosols, and lyophilized formulations. Depending on route of administration used, special devices may be required for application or administration of the drug, such as, for example, syringes and needles, inhalers, pumps, injection pens, applicators, or special flasks. Pharmaceutical dosage forms are often composed of the drug, an excipient(s), and a container/closure system. One or multiple excipients, also referred to as inactive ingredients, can be added to an anti-CTGF agent to improve or facilitate manufacturing, stability, administration, and safety of the agent, and can provide a means to achieve a desired agent release profile. Therefore, the type of excipient(s) to be added to the agent can depend on various factors, such as, for example, the physical and chemical properties of the agent, the route of administration, and the manufacturing procedure. Pharmaceutically acceptable excipients are available in the art, and include those listed in various pharmacopoeias. (See, e.g., USP, JP, EP, and BP), Inactive Ingredient Guide available through the FDA's website, and Handbook of Pharmaceutical Additives, ed. Ash; Synapse Information Resources, Inc. (2002))

Pharmaceutical dosage forms of a compound for use in the present invention may be manufactured by any of the methods well-known in the art, such as, for example, by conventional mixing, sieving, dissolving, melting, granulating, dragee-making, tabletting, suspending, extruding, spray-drying, levigating, emulsifying, (nano/micro-) encapsulating, entrapping, or lyophilization processes. As noted above, the compositions for use in the present invention can include one or more physiologically acceptable inactive ingredients that facilitate processing of active molecules into preparations for pharmaceutical use.

Proper formulation is dependent upon the desired route of administration. For intravenous injection, for example, the composition may be formulated in aqueous solution, if necessary, using physiologically compatible buffers, including, for example, phosphate, histidine, or citrate for adjustment of the formulation pH, and a tonicity agent, such as, for example, sodium chloride or dextrose. For transmucosal or nasal administration, semisolid, liquid formulations, or patches may be preferred, possibly containing penetration enhancers. Such penetrants are generally known in the art. For oral administration, the compounds can be formulated in liquid or solid dosage forms and as instant or controlled/sustained release formulations. Suitable dosage forms for oral ingestion by a subject include tablets, pills, dragees, hard and soft shell capsules, liquids, gels, syrups, slurries, suspensions, and emulsions.

Solid oral dosage forms can be obtained using excipients, which may include, fillers, disintegrants, binders (dry and wet), dissolution retardants, lubricants, glidants, antiadherants, cationic exchange resins, wetting agents, antioxidants, preservatives, coloring, and flavoring agents. These excipients can be of synthetic or natural source. Examples of such excipients include cellulose derivatives, citric acid, dicalcium phosphate, gelatine, magnesium carbonate, magnesium/sodium lauryl sulfate, mannitol, polyethylene glycol, polyvinyl pyrrolidone, silicates, silicium dioxide, sodium benzoate, sorbitol, starches, stearic acid or a salt thereof, sugars (i.e. dextrose, sucrose, lactose, etc.), talc, tragacanth mucilage, vegetable oils (hydrogenated), and waxes. Ethanol and water may serve as granulation aides. In certain instances, coating of tablets with, for example, a taste-masking film, a stomach acid resistant film, or a release-retarding film is desirable. Natural and synthetic polymers, in combination with colorants, sugars, and organic solvents or water, are often used to coat tablets, resulting in dragees. When a capsule is preferred over a tablet, the drug powder, suspension, or solution thereof can be delivered in a compatible hard or soft shell capsule.

Compositions formulated for parenteral administration by injection are usually sterile and, can be presented in unit dosage forms, e.g., in ampoules, syringes, injection pens, or in multi-dose containers, the latter usually containing a preservative. The compositions may take such forms as suspensions, solutions, or emulsions in oily or aqueous vehicles, and may contain formulatory agents, such as buffers, tonicity agents, viscosity enhancing agents, surfactants, suspending and dispersing agents, antioxidants, biocompatible polymers, chelating agents, and preservatives. Depending on the injection site, the vehicle may contain water, a synthetic or vegetable oil, and/or organic co-solvents. In certain instances, such as with a lyophilized product or a concentrate, the parenteral formulation would be reconstituted or diluted prior to administration. Depot formulations, providing controlled or sustained release of an anti-CTGF agent, may include injectable suspensions of nano/micro particles or nano/micro or non-micronized crystals. Polymers such as poly(lactic acid), poly(glycolic acid), or copolymers thereof, can serve as controlled/sustained release matrices, in addition to others well known in the art. Other depot delivery systems may be presented in form of implants and pumps requiring incision.

Suitable carriers for intravenous injection for the molecules of the invention are well-known in the art and include water-based solutions containing a base, such as, for example, sodium hydroxide, to form an ionized compound, sucrose or sodium chloride as a tonicity agent, for example, the buffer contains phosphate or histidine. Co-solvents, such as, for example, polyethylene glycols, may be added. These water-based systems are effective at dissolving compounds of the invention and produce low toxicity upon systemic administration. The proportions of the components of a solution system may be varied considerably, without destroying solubility and toxicity characteristics. Furthermore, the identity of the components may be varied. For example, low-toxicity surfactants, such as polysorbates or poloxamers, may be used, as can polyethylene glycol or other co-solvents, biocompatible polymers such as polyvinyl pyrrolidone may be added, and other sugars and polyols may substitute for dextrose.

Anti-CTGF antibody formulations for use in accordance with the present invention may be prepared by mixing an anti-CTGF antibody with pharmaceutically acceptable carriers, excipients or stabilizers that are nontoxic to recipients at the dosages and concentrations employed. Anti-CTGF antibody formulations may include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); carriers; hydrophilic polymers such as polyvinylpyrrolidone; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes; and/or non-ionic surfactants or polyethylene glycol.

In particular, anti-CTGF antibody formulations may further comprise low molecular weight polypeptides; carriers such as serum albumin, gelatin, or immunoglobulins; and amino acids such as glycine, glutamine, asparagine, histidine, arginine, or lysine. The anti-CTGF antibody formulations can be lyophilized as described in PCT/US 1996/012251. Additionally, sustained-release preparations may also be prepared. Frequently, polymers such as poly(lactic acid), poly(glycolic acid), or copolymers thereof serve as controlled/sustained release matrices, in addition to others well known in the art.

The anti-CTGF antibodies can be supplied or administered at any desired concentration. In some embodiments, the anti-CTGF antibody concentration is at least 1 mg/ml, 5 mg/ml, 10 mg/ml, 20 mg/ml, 25 mg/ml, 50 mg/ml, 75 mg/ml, 100 mg/ml, 125 mg/ml, 150 mg/ml, or 200 mg/ml. In other embodiments, the anti-CTGF antibody concentration is no more than about 5 mg/ml, 10 mg/ml, 20 mg/ml, 25 mg/ml, 50 mg/ml, 75 mg/ml, 100 mg/ml, 125 mg/ml, 150 mg/ml, 200 mg/ml, 250 mg/ml, or 300 mg/ml. In further embodiments, the anti-CTGF antibody concentration is between 5 mg/ml to 20 mg/ml, 20 mg/ml to 50 mg/ml, 50 mg/ml to 100 mg/ml, 100 mg/ml to 200 mg/ml, or 200 mg/ml to 300 mg/ml.

**Articles of Manufacture**

The present compositions may, if desired, be presented in a pack or dispenser device containing one or more unit dosage forms containing an anti-CTGF agent. Such a pack or device may, for example, comprise metal or plastic foil, glass and rubber stoppers, vials or syringes. The container holding an anti-CTGF agent composition that is effective for treating a MD and may have a sterile access port (for example the container may be an intravenous solution bag or a vial having a stopper pierceable by a hypodermic injection needle). The article of manufacture may further comprise an additional container comprising a pharmaceutically acceptable diluent buffer, such as bacteriostatic water for injection (BWFI), phosphate-buffered saline, Ringer's solution, and/or dextrose solution. The article of manufacture may further include other materials desirable from a commercial and user standpoint, including, for example, filters or needles.

Compositions comprising an anti-CTGF agent formulated in a compatible pharmaceutical carrier may be provided in an appropriate container that is labeled for treatment of a MD. The pack or dispenser device may be accompanied by instructions for administration that provide specific guidance regarding dosing the anti-CTGF agent including a description of the type of patients who may be treated (e.g., a person with DMD), the schedule (e.g., dose and frequency) and route of administration, and the like.

These and other embodiments of the present invention will readily occur to those of ordinary skill in the art in view of the disclosure herein.

**EXAMPLES**

The invention will be further understood by reference to the following examples, which are intended to be purely exemplary of the invention. These examples are provided solely to illustrate the claimed invention. The present invention is not limited in scope by the exemplified embodiments, which are intended as illustrations of single aspects of the invention only. Any methods that are functionally equivalent are within the scope of the invention. Various modifications of the invention in addition to those described herein will become apparent to those skilled in the art from the foregoing description and accompanying figures. Such modifications are intended to fall within the scope of the appended claims.

An ongoing open-label, single-arm Phase 2 clinical study was analyzed for improvements in pulmonary, cardiac, and muscle function endpoints, relative to a historical control(s), after all enrolled patients completed their Week 52 visit. The enrollment criteria included a subject age greater than twelve years of age, non-ambulatory status and receiving standard of care.

The median age of subjects was 15.8 years (12.4, 25.6). All subjects were male and the majority were white (20 Caucasians and 1 Asian). The most common reported medical history were femur fracture (33.3%), restrictive lung disease (29%), headache/migraine (29%), scoliosis (24%), tenotomy (19%), asthenia (19%), sleep apnea (19%) and cardiomyopathy (14.5%). Median age at DMD diagnosis was 5.5 years (0.6, 12.2), with a median duration of being non-ambulatory prior to study enrollment of 3.4 years (1, 11.5). The majority of subjects had dystrophin gene deletion (57%), while the rest of subjects had duplication and point mutations. All subjects are on corticosteroids (deflazacort 43%, prednisone 57%). Corticosteroids started approximately (median) 6 years (3,0, 17) prior to enrollment in the study. Subjects were treated with pamrevlumab (FibroGen, Inc., San Francisco, CA) at a dose of 35 mg/kg using i.v. infusion administered at two week intervals and had completed 52 weeks of treatment of a planned treatment course of 156 weeks. A total of 21 non-ambulatory subjects are enrolled in the study and are undergoing treatment with pamrevlumab.

**Example 1: Pamrevulmab Treatment Improves Pulmonary Function**

Pulmonary dysfunction in DMD is progressive and restrictive in nature. Additionally, it is one of the most common causes of morbidity and mortality in non-ambulatory patients. Forced vital capacity assessed by spirometry, is the best global assessment of all respiratory muscles, because it requires a full inspiration (reflecting function of inspiratory muscles) and a full expiration (reflecting function of expiratory muscles) (Finder et al. *supra*). Pulmonary function tests have an excellent feasibility (defined as the percent of subjects able to perform the task) of > 95% in non-ambulatory DMD patients (Connolly et al. Muscle Nerve 2015;51(4):522-532).

Three separate studies have shown that once they are in the decline phase, both steroid and non-steroid treated DMD patients have a similar rate of decline in pulmonary function (Mayer et al., *supra*; Connolly et al., *supra*; McDonald et al. Parent Project Muscular Dystrophy Pulmonary Endpoints Workshop. April 14, 2016, Bethesda, MD).

Considering the historical published data above, at Week 48, a remarkable 26% of the subjects in the current study had an improvement or showed no decline in FVCpp compared to their baseline measurement (Figure 1). The LS estimate of the mean change from baseline in FVCpp at 1-year was -4.00% (Figure 2). This change from baseline in FVCpp of pamrevlumab treated subjects compares favorably to the decline in FVCpp seen in non-ambulatory subjects over the natural course of the disease, -5.47%, p = 0.1344 (Ricotti et al. *supra)* (Figure 2).

Another pulmonary function measurement is FEV1. This test assesses airway function and is the standard global assessment in both clinical and research studies of asthma, chronic obstructive pulmonary disease, and cystic fibrosis. With exception of chronic obstructive pulmonary disease, FEV1 very closely follows FVC with the advantage of requiring less prolonged effort (1 second) (Finder et al., *supra).*

In the current study, at Week 48, 22% of the subjects, importantly, had an improvement or showed no decline in FEV1 % predicted (FEV1pp) compared to their baseline measurement (Figure 3). The LS estimate of the mean change from baseline at 1-year in FEVlpp was -4.53% calculated from 18 subjects who underwent FEV1 testing. The current study's data compares favorably to the data from both a placebo group and a glucocorticosteroid treated group that showed yearly rates of FEVlpp decline of -10.2% and -8.7, respectively, p =0.0542 (Meier et al., *supra*) (Figure 4).

Pulmonary function was further tested using an expiratory flow test because this type of test is easily measured and the results predominantly reflect expiratory muscle function. Peak Expiratory Flow (PEF) rate was chosen as it requires full inspiration to achieve maximal flow and therefore PEF rate measures both inspiratory and expiratory muscle function (Finder et al., *supra*).

In the current study, at Week 48, a notable 52% of the subjects had an improvement or showed no decline in PEFRpp from baseline compared to their baseline measurement (Figure 5). The LS estimate of the mean change from baseline at 1-year in PEFRpp was -2.10% (Figure 6). In contrast, the annual rate of change for PEFRpp based on historic data in non-ambulatory DMD patients was -4.81%, p = 0.1842, (Ricotti et al. *supra)* (Figure 6).

**Example 2: Pamrevlumab Treatment Improves Cardiac Function**

Patients with DMD, have progressive cardiac muscle dysfunction, which is the leading cause of mortality. Care considerations for DMD state that pharmacological therapy should be initiated with the onset of heart failure symptoms or when abnormalities such as depressed LVEF, abnormal chamber dimensions, or the presence of myocardial fibrosis are noted on imaging studies (CMR or echocardiogram) (Birnkrant et al. Lancet Neurol. 2018;17(4):347-361).

In the current study, cardiac MRI was used to assess LVEF at 1-year. Impressively, it was found that 47% of subjects had an improvement or showed no decline in LVEF (Figure 7). The LS estimate of the mean change from baseline in LVEF at 1-year is +0.2911%. These results are remarkable in light of the progression seen over the natural disease history, -0.82000%, p = 0.1210, observed in historic controls (McDonald et al. 2018, *supra)* (Figure 8).

When the more sensitive technique of serial analysis of left ventricular myocardial peak circumferential strain ($\varepsilon_{cc}$) was employed to evaluate cardiac function, an even more impressive 57% of the subjects had improvement in cardiac function by circumferential peak strain (Figure 9).

When the change from baseline at 1-year in the current study was analyzed for global circumferential strain, 64% of the subjects had an improvement in cardiac function (Figure 10). The mean change from baseline in global circumferential strain (%) for the current subjects was 1.7433, treated with pamrevubmab.

Progressive myocardial fibrosis was additionally monitored by mass late gadolinium enhancement (MLGE). Consistent with the effects observed above, MLGE assessment showed an impressive reduction or stabilization in cardiac fibrosis score in 50% of the subjects (Figure 11). The estimated mean change from baseline for this group at 1-year was - 1.17g.

A scatter plot and Spearman correlation analysis of the change from baseline to 1-year of LVEF (%) compared to change in cardiac fibrosis score (MLGE) at 1-year demonstrates that the reduction in cardiac fibrosis score correlates with an increase in LVEF(%) (Figure 12).

**Example 3: Pamrevlumab Treatment Improves Skeletal Muscle Function**

Preserving upper limb strength is very important in non-ambulatory DMD patients, because it provides functional independence. Hand held myometry (HHM) was used in the current study to measure distal upper arm strength (grip strength), and it has shown very good reliability and feasibility in non-ambulatory DMD patients (Connolly et al., *supra*). Several studies have shown that the change in hand grip strength over 1-year decreases in non-ambulatory DMD patients (Hogrel et al., *supra*; Seferian et al. *supra*). For example, Seferian et al. demonstrated that over the course of one-year, non-ambulatory DMD patients showed a mean decline in grip strength of (-3.0 newton, non-dominant arm; -2.7 newton dominant arm).

In the current study, a remarkable 32% and 58% of subjects had an increase or showed no decline in grip strength, respectively, of their dominant hand (Figure 13A) and non-dominant hand (Figure 13B). The LS estimate of the mean change from baseline in grip strength in the dominant hand at 1-year was +3.34 newton (p = 0.0553), while in the non-dominant hand was +3.35 newton (p = 0.0346). (Figure 14) These results are noteworthy in light of the decline in grip strength from baseline seen in Seferian et al. over the natural course of the disease in non-ambulatory DMD patients, -2.7 newton, dominant hand, and -3.0 newton, non-dominant hand, (Seferian et al., *supra*) (Figure 14).

The PUL assessment, developed specifically for DMD, is an additional method of assessing upper extremity function in both ambulant and non-ambulant individuals (Mayhew et al. Dev Med Child Neurol. 2013;55(11):1038-45; Pane M et al. Neuromuscul Disord. 2014;24(3):201-6). This test provides a total upper extremity functional score capable of characterizing overall progression and severity of disease, while its component subscores assess shoulder-, middle-, and distal-level function allowing the tracking of proximal-to-distal progressive loss of upper limb function. In the current study, 19 subjects were assessed by PUL at Week 48 following treatment with pamrevlumab. Encouragingly, 42% of the subjects showed an improvement or showed no decline in PUL score compared to a baseline measurement. (Figure 15) The LS estimate of the mean change from baseline at 1-year in PUL score was -1.53 and compares favorably to the PUL score (-4.13) seen in Rocotti et al. over the natural course of the disease in non-ambulatory DMD patients (Rocotti et al. *supra*) (Figure 16).

In addition to strength testing, the degree of inflammation, edema, dystrophic muscle composition and fat infiltration of the upper arms (biceps brachii) were assessed through T2-mapping using MRI and CAD assessment (Hogrel JY et al. *supra*). Previous studies have demonstrated that the percentage of fat in the upper limb muscles is highly correlated with functional assessments and increases linearly with age. In the current study, 11 subjects had their upper arms assessed by MRI at 1-year following treatment with pamrevlumab. A remarkable 81% of the subjects had a reduction or showed no change in inflammation, edema and dystrophic muscle composition as evidenced by their T2-mapping score (Figure 17). When the mean change from baseline at 1-year for the fat fraction (%) score was examined, it was observed that the mean change was markedly reduced, 0.6%, compared to 3.2% seen in Hogrel et al. showing the natural course of the disease in non-ambulatory DMD patients (Hogrel et al., *supra*) (Figure 18).

A scatter plot and Spearman correlation of changes from baseline at 1-year in PUL total score compared to change at 1-year in upper arm biceps brachii T2-mapping demonstrates that the increase in PUL score correlates with a reduction in upper arm inflammation, edema, dystrophic muscle composition and fat infiltration (Figure 19).

In sum, the results described above demonstrated that the methods and agents of the invention were effective in improving pulmonary function, improving cardiac function, and maintaining skeletal muscle strength, key organ systems affected by DMD. Further, these results show that methods and agents of the invention were effective for reducing muscle inflammation, edema and fat infiltration. These findings demonstrate that inhibition of CTGF, using the methods and agents of the invention, provide an effective treatment to ameliorate symptoms of DMD and other forms of MDs.

Various modifications of the invention, in addition to those shown and described herein, will become apparent to those skilled in the art from the foregoing description. Such modifications are intended to fall within the scope of the appended claims.

All references cited herein are hereby incorporated by reference herein in their entirety.

Further numbered embodiments:

1. A method of improving pulmonary function in a subject with a muscular dystrophy, the method comprising administering to a subject in need thereof an effective amount of an agent that inhibits connective tissue growth factor (CTGF), thereby improving the subject's pulmonary function.

2. The method of embodiment 1, wherein the muscular dystrophy is selected from the group consisting of Duchenne muscular dystrophy, Becker's muscular dystrophy, congenital muscular dystrophy, distal muscular dystrophy, Emery-Dreifuss muscular dystrophy, facioscapulohumeral muscular dystrophy, limb-girdle muscular dystrophy, myotonic muscular dystrophy, and oculopharyngeal muscular dystrophy.

3. The method of embodiment 2, wherein the muscular dystrophy is Duchenne muscular dystrophy.

4. The method of embodiment 1, wherein the subject is non-ambulatory.

5. The method of embodiment 1, wherein the improvement in pulmonary function is a reduction in the rate of decline, a stabilization in the rate of decline or a reversal (improvement) in the rate of decline of the subject's forced vital capacity % predicted (FVCpp), forced expiratory volume % predicted at 1 second (FEV1 pp), peak expiratory flow rate % predicted (PEFRpp), maximum static inspiratory pressure % predicted (MIPpp) or maximum static expiratory pressure % predicted (MEPpp).

6. The method of embodiment 1, wherein the anti-CTGF agent is selected from group consisting of anti-CTGF antibodies, anti-CTGF antibody fragments, anti-CTGF antibody mimetics and anti-CTGF oligonucleotides.

7. The method of embodiment 6, wherein the anti-CTGF agent is an anti-CTGF antibody.

8. The method of embodiment 7, wherein the anti-CTGF antibody is a human or humanized antibody.

9. The method of embodiment 7, wherein the anti-CTGF antibody is pamrevlumab.

10. The method of embodiment 7, wherein the anti-CTGF antibody binds to CTGF competitively with pamrevulmab.

11. The method of embodiment 7, wherein the effective amount of an anti-CTGF antibody is at least 35 mg/kg.

12. The method embodiment 6, wherein the anti-CTGF oligonucleotide is selected from the group consisting of antisense oligonucleotides, siRNAs, miRNAs and shRNAs.

13. The method of embodiment 1, further comprising the administration of a corticosteroid, ataluren and/or eteplirsen.

14. A method of improving cardiac function in a subject with a muscular dystrophy, the method comprising administering to a subject in need thereof an effective amount of an agent that inhibits connective tissue growth factor (CTGF), thereby improving the subject's cardiac function.

15. The method of embodiment 14, wherein the improvement in cardiac function is a reduction in the rate of decline, a stabilization in the rate of decline or a reversal (improvement) in the rate of decline of the subject's left ventricular ejection fraction percentage (LVEF%), circumferential peak strain (%), or global circumferential strain (%).

16. A method of reducing the development of or reducing the progression rate of cardiac fibrosis in a subject with a muscular dystrophy, the method comprising administering to a subject in need thereof an effective amount of an anti-CTGF agent, thereby reducing the development of or reducing the progression rate of cardiac fibrosis in the subject.

17. The method of embodiment 16, wherein the subject's degree of cardiac fibrosis is assessed by mass of late gadolinium enhancement.

18. A method of increasing the muscle strength of a subject with a muscular dystrophy, the method comprising administering to a subject in need thereof an effective amount of an anti-CTGF agent, thereby increasing the subject's muscle strength.

19. The method of embodiment 18, wherein the increased muscle strength is measured as an increase in the subject's grip strength.

20. A method for reducing muscle inflammation, muscle edema, fat infiltration or percentage of dystrophic muscle composition in a subject with a muscular dystrophy, the method comprising administering to the subject an effective amount of an anti-CTGF agent, thereby reducing muscle inflammation, muscle edema, fat infiltration or percentage of dystrophic muscle composition in the subject.

21. The method of any one of embodiments 1, 14, 16, 18 or 20, wherein the subject is non-ambulatory.

## Claims

**1.** An agent that inhibits connective tissue growth factor (CTGF) for use in a method of improving pulmonary function in a subject with a muscular dystrophy, the method comprising administering to a subject in need thereof an effective amount of the agent, thereby improving the subject's pulmonary function.

**2.** The agent for use according to claim 1, wherein the muscular dystrophy is selected from the group consisting of Duchenne muscular dystrophy, Becker's muscular dystrophy, congenital muscular dystrophy, distal muscular dystrophy, Emery-Dreifuss muscular dystrophy, facioscapulohumeral muscular dystrophy, limb-girdle muscular dystrophy, myotonic muscular dystrophy, and oculopharyngeal muscular dystrophy.

**3.** The agent for use according to claim 2, wherein the muscular dystrophy is Duchenne muscular dystrophy.

**4.** The agent for use according to any of claims 1-3, wherein:

(a) the subject is non-ambulatory; and/or
(b) the improvement in pulmonary function is a reduction in the rate of decline, a stabilization in the rate of decline or a reversal (improvement) in the rate of decline of the subject's forced vital capacity % predicted (FVCpp), forced expiratory volume % predicted at 1 second (FEV1 pp), peak expiratory flow rate % predicted (PEFRpp), maximum static inspiratory pressure % predicted (MIPpp) or maximum static expiratory pressure % predicted (MEPpp).

**5.** The agent for use according to any of claims 1-4, wherein (c) the anti-CTGF agent is selected from group consisting of anti-CTGF antibodies, anti-CTGF antibody fragments, anti-CTGF antibody mimetics and anti-CTGF oligonucleotides.

**6.** The agent for use according to claim 5, wherein the anti-CTGF agent is an anti-CTGF antibody.

**7.** The agent for use according to claim 6, wherein:

(a) the anti-CTGF antibody is a human or humanized antibody;
(b) the anti-CTGF antibody is pamrevlumab;
(c) the anti-CTGF antibody binds to CTGF competitively with pamrevulmab; and/or
(d) the effective amount of an anti-CTGF antibody is at least35 mg/kg.

**8.** The agent for use according to claim 5, wherein the anti-CTGF oligonucleotide is selected from the group consisting of antisense oligonucleotides, siRNAs, miRNAs and shRNAs.

**9.** The agent for use according to any of claims 1-8, further comprising the administration of a corticosteroid, ataluren and/or eteplirsen.

**10.** An agent that inhibits connective tissue growth factor (CTGF) for use in a method of improving cardiac function in a subject with a muscular dystrophy, the method comprising administering to a subject in need thereof an effective amount of the agent, thereby improving the subject's cardiac function.

**11.** The agent for use according to claim 10, wherein the improvement in cardiac function is a reduction in the rate of decline, a stabilization in the rate of decline or a reversal (improvement) in the rate of decline of the subject's left ventricular ejection fraction percentage (LVEF%), circumferential peak strain (%), or global circumferential strain (%).

**12.** An anti-CTGF agent for use in a method of reducing the development of or reducing the progression rate of cardiac fibrosis in a subject with a muscular dystrophy, the method comprising administering to a subject in need thereof an effective amount of the anti-CTGF agent, thereby reducing the development of or reducing the progression rate of cardiac fibrosis in the subject.

**13.** The agent for use according to claim 12, wherein the subject's degree of cardiac fibrosis is assessed by mass of late gadolinium enhancement.

**14.** An anti-CTGF agent for use in a method of increasing the muscle strength of a subject with a muscular dystrophy, the method comprising administering to a subject in need thereof an effective amount of the anti-CTGF agent, thereby increasing the subject's muscle strength.

**15.** The agent for use according to claim 14, wherein the increased muscle strength is measured as an increase in the subject's grip strength.

**16.** An anti-CTGF agent for use in a method for reducing muscle inflammation, muscle edema, fat infiltration or percentage of dystrophic muscle composition in a subject with a muscular dystrophy, the method comprising administering to the subject an effective amount of the anti-CTGF agent, thereby reducing muscle inflammation, muscle edema, fat infiltration or percentage of dystrophic muscle composition in the subject.

**17.** The agent for use according to any one of claims 1-16, wherein the subject is non-ambulatory.

Waterfall Plot of Change from Baseline at Week 48 in FVCpp

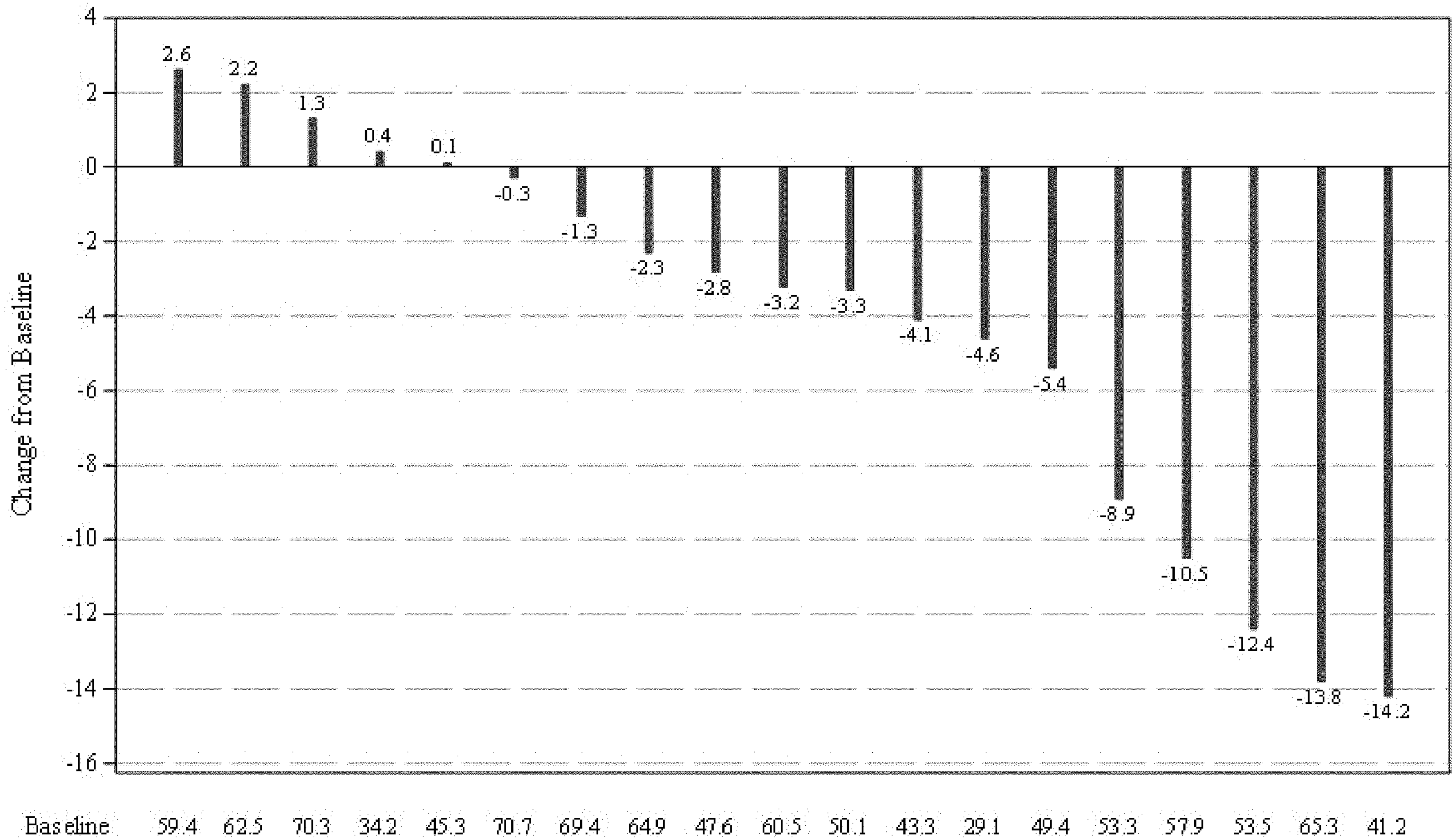


**Fig. 1**

Fig. 2 Bar Plot of LS Mean Change from Baseline at 1 Year in FVCpp

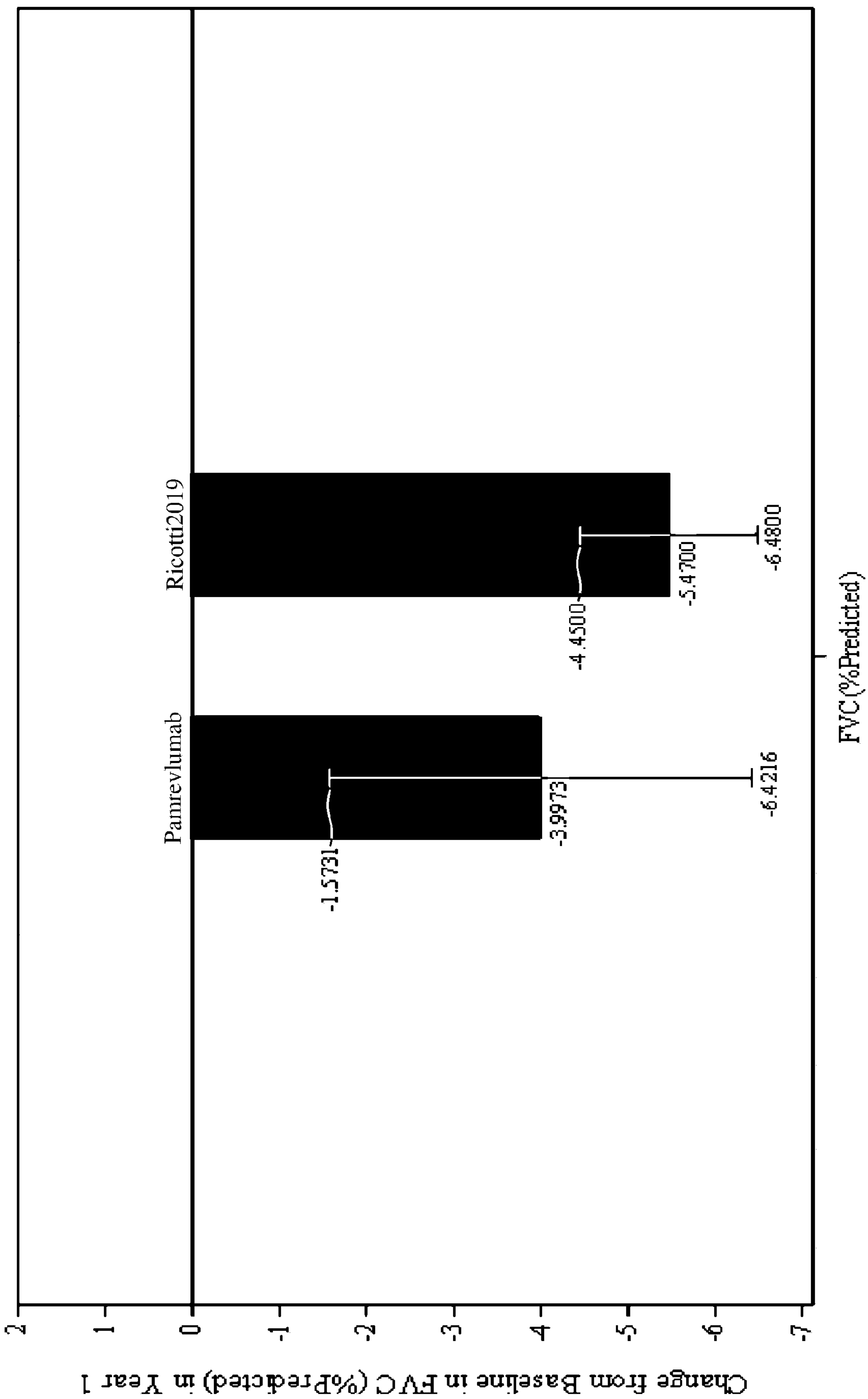

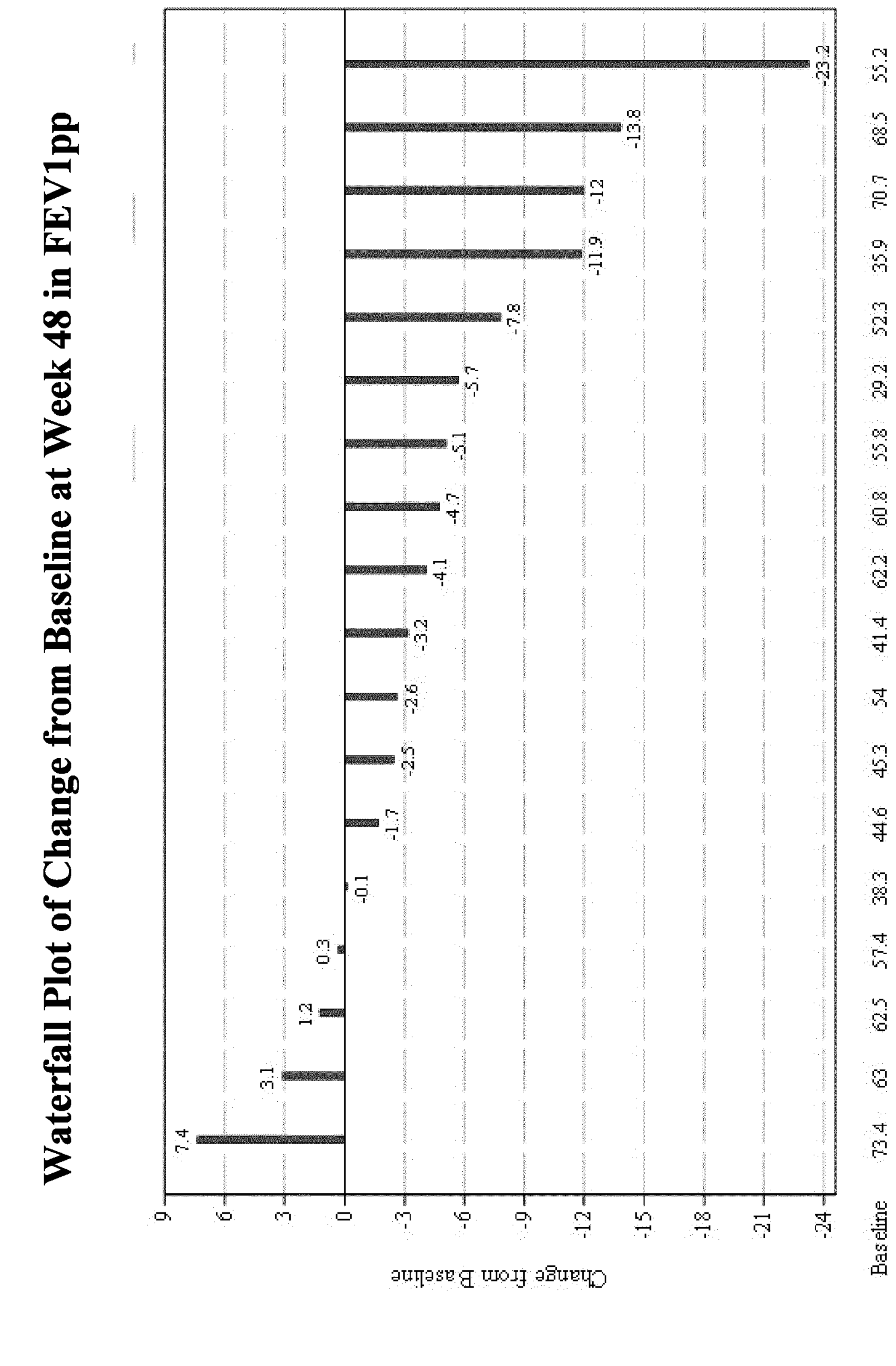
Waterfall Plot of Change from Baseline at Week 48 in FEV1pp
Change from Baseline
9
6
3
0
-3
-6
-9
-12
-15
-18
-21
-24
Baseline
73.4
63
62.5
57.4
38.3
44.6
45.3
54
41.4
62.2
60.8
55.8
29.2
52.3
35.9
70.7
68.5
55.2
7.4
3.1
1.2
0.3
-0.1
-1.7
-2.5
-2.6
-3.2
-4.1
-4.7
-5.1
-5.7
-7.8
-11.9
-12
-13.8
-23.2

Fig. 3

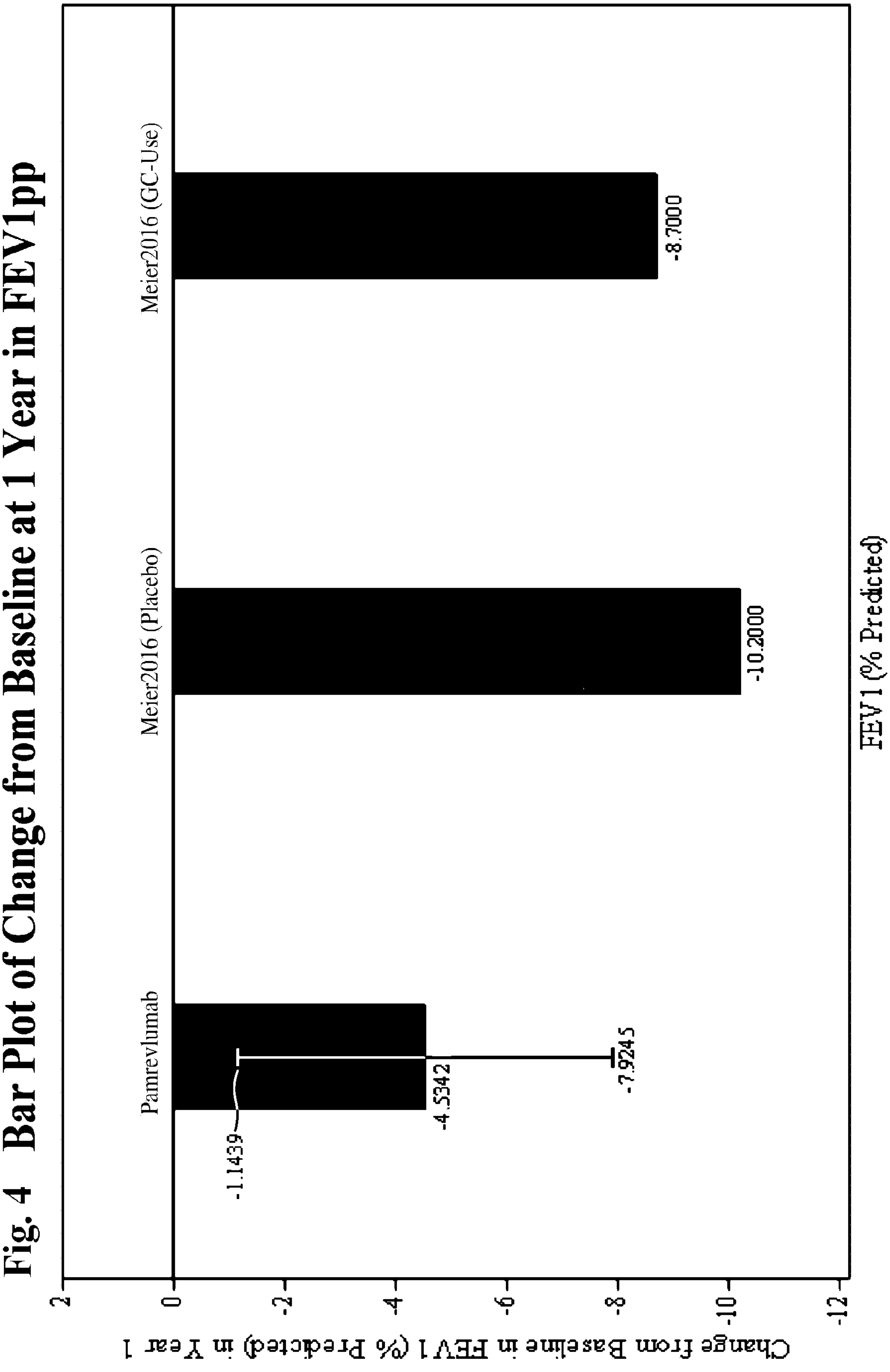


**Fig. 4 Bar Plot of Change from Baseline at 1 Year in FEV1pp**

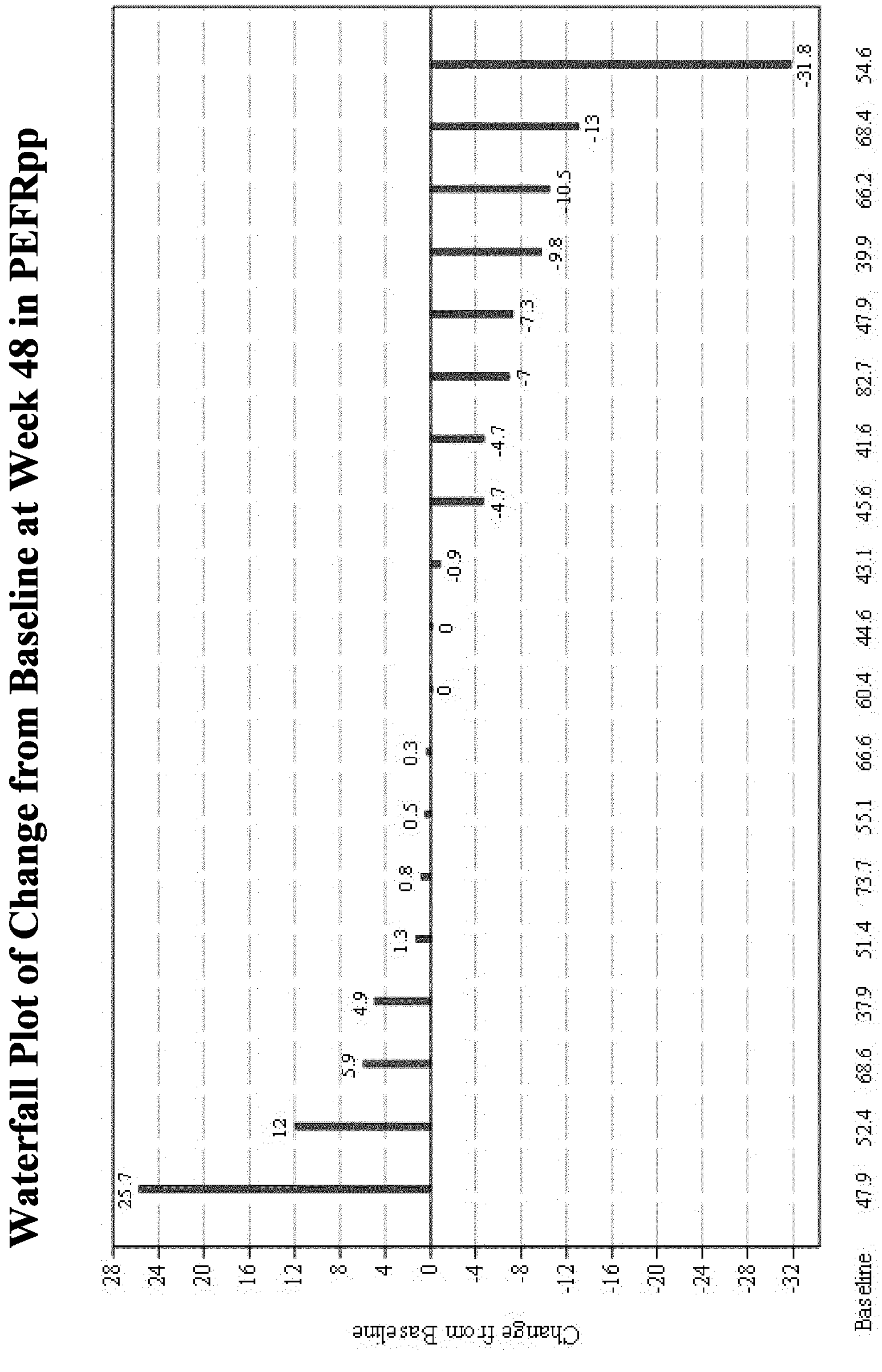

Waterfall Plot of Change from Baseline at Week 48 in PEFRpp
Change from Baseline
28
24
20
16
12
8
4
0
-4
-8
-12
-16
-20
-24
-28
-32
Baseline
47.9
52.4
68.6
37.9
51.4
73.7
55.1
66.6
60.4
44.6
43.1
45.6
41.6
82.7
47.9
39.9
66.2
68.4
54.6
25.7
12
5.9
4.9
1.3
0.8
0.5
0.3
0
0
-0.9
-4.7
-4.7
-7
-7.3
-9.8
-10.5
-13
-31.8


Fig. 5

Fig. 6 Bar Plot of Change from Baseline at 1 Year in PEFRpp

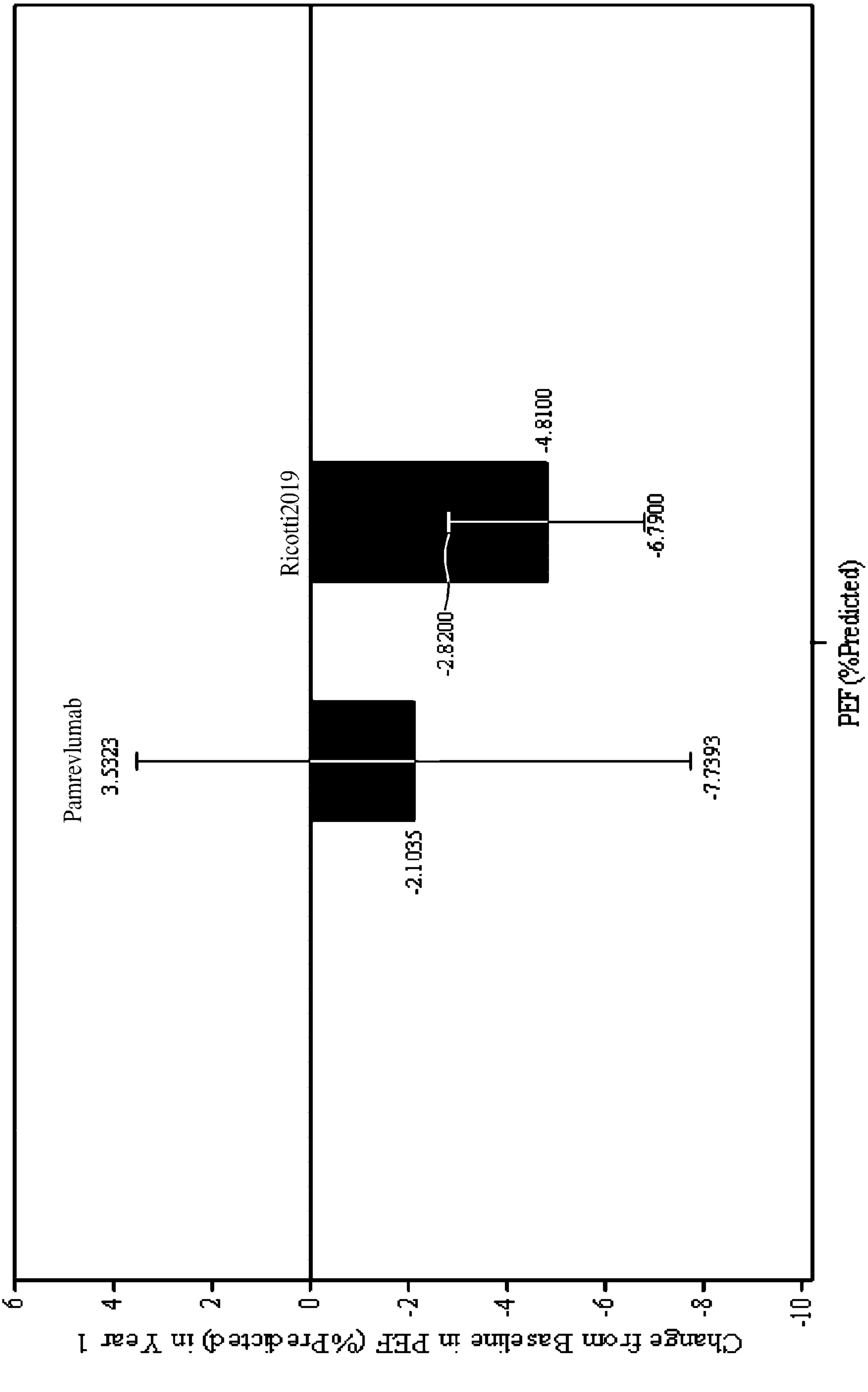

**Waterfall Plot of Change from Baseline at 1 Year in LVEF**

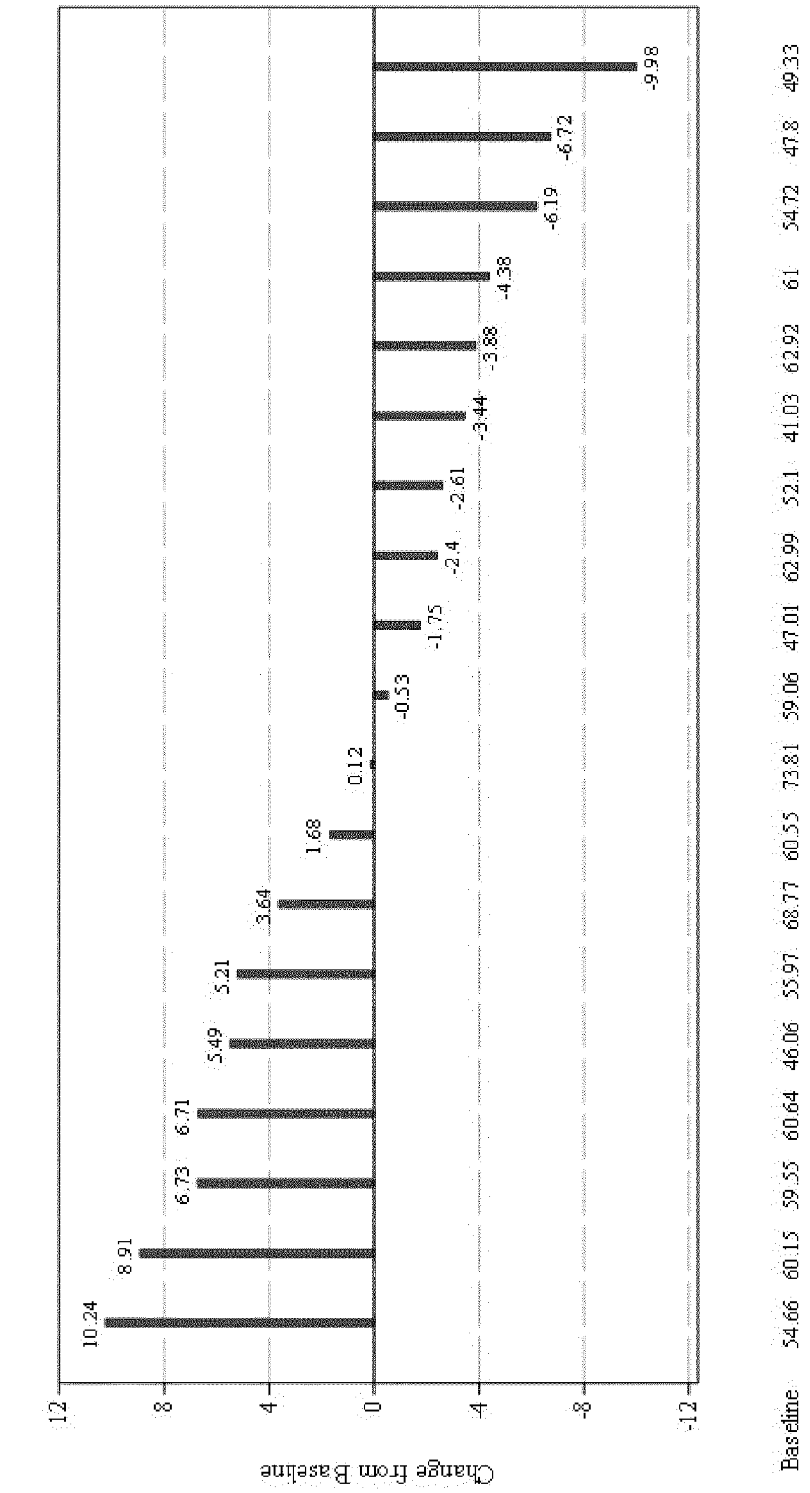


Fig. 7

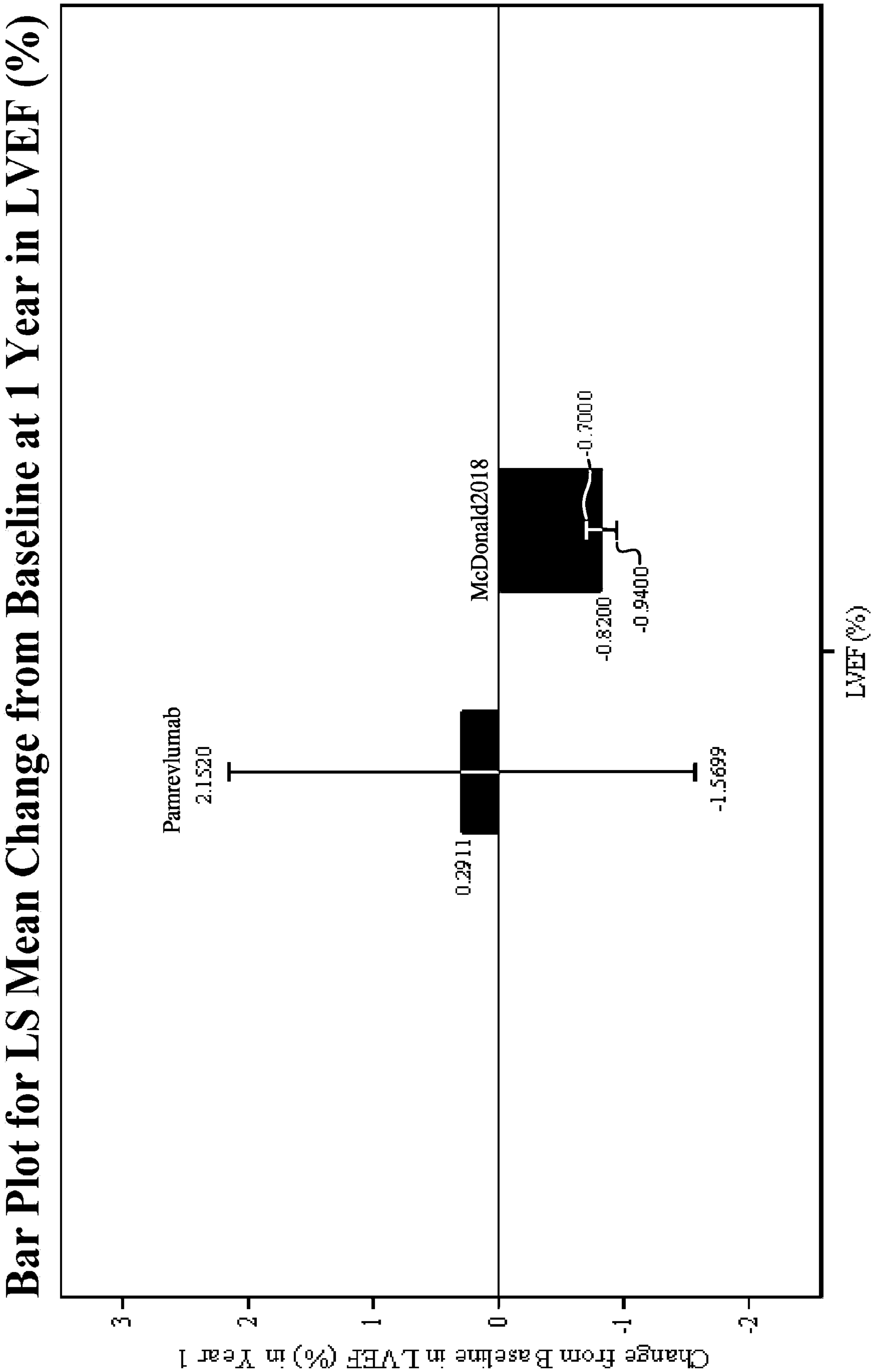
Bar Plot for LS Mean Change from Baseline at 1 Year in LVEF (%)
Pamrevlumab
2.1520
0.2911
-1.5699
McDonald2018
-0.7000
-0.8200
-0.9400
3
2
1
0
-1
-2
Change from Baseline in LVEF (%) in Year 1
LVEF (%)

Fig. 8

**Waterfall Plot of Change from Baseline at 1 Year in Mean Circumferential Peak Strain (%)**

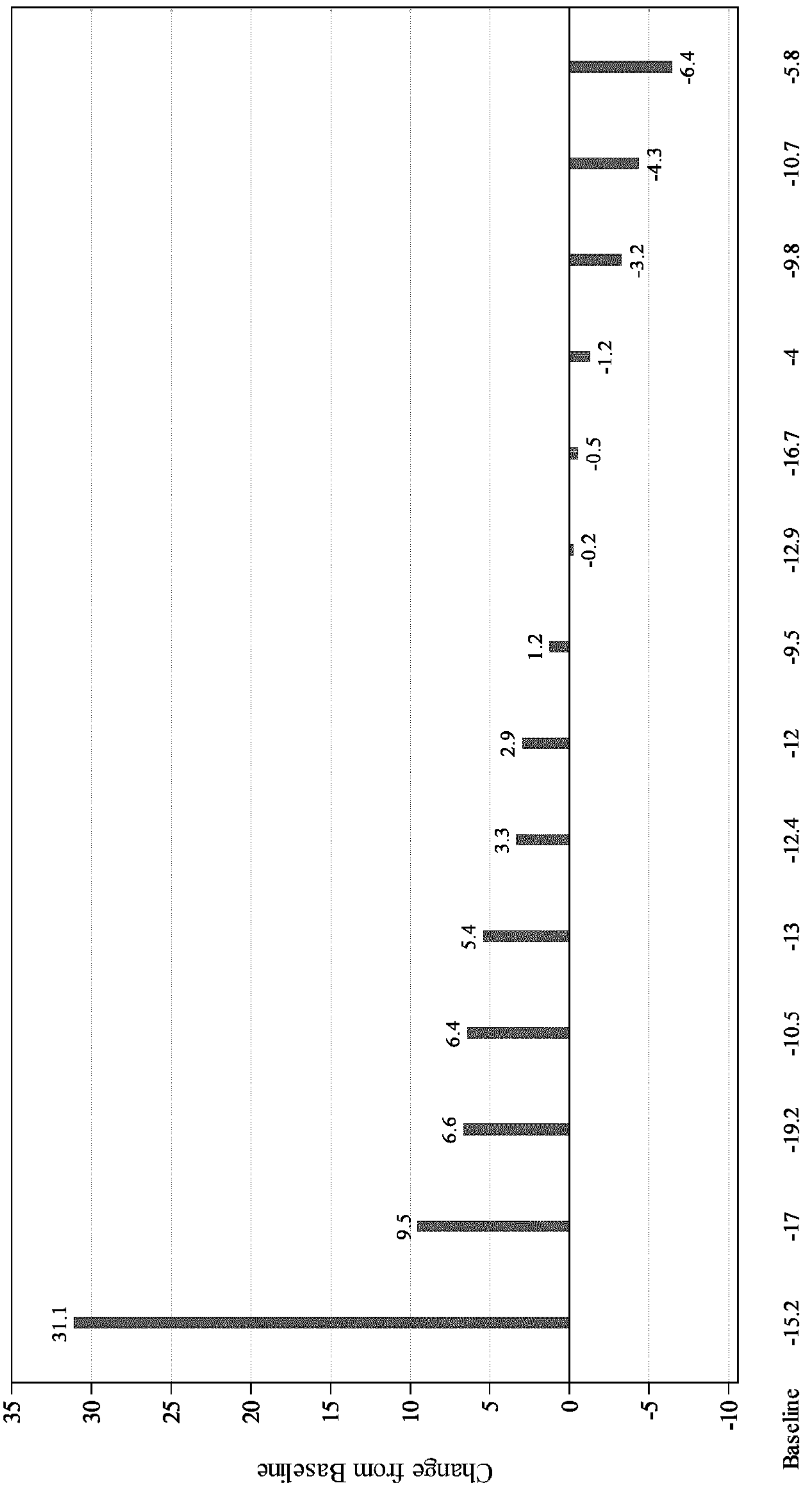


**Fig. 9**

**Waterfall Plot of Change from Baseline at 1 Year in Global Circumferential Strain (%)**

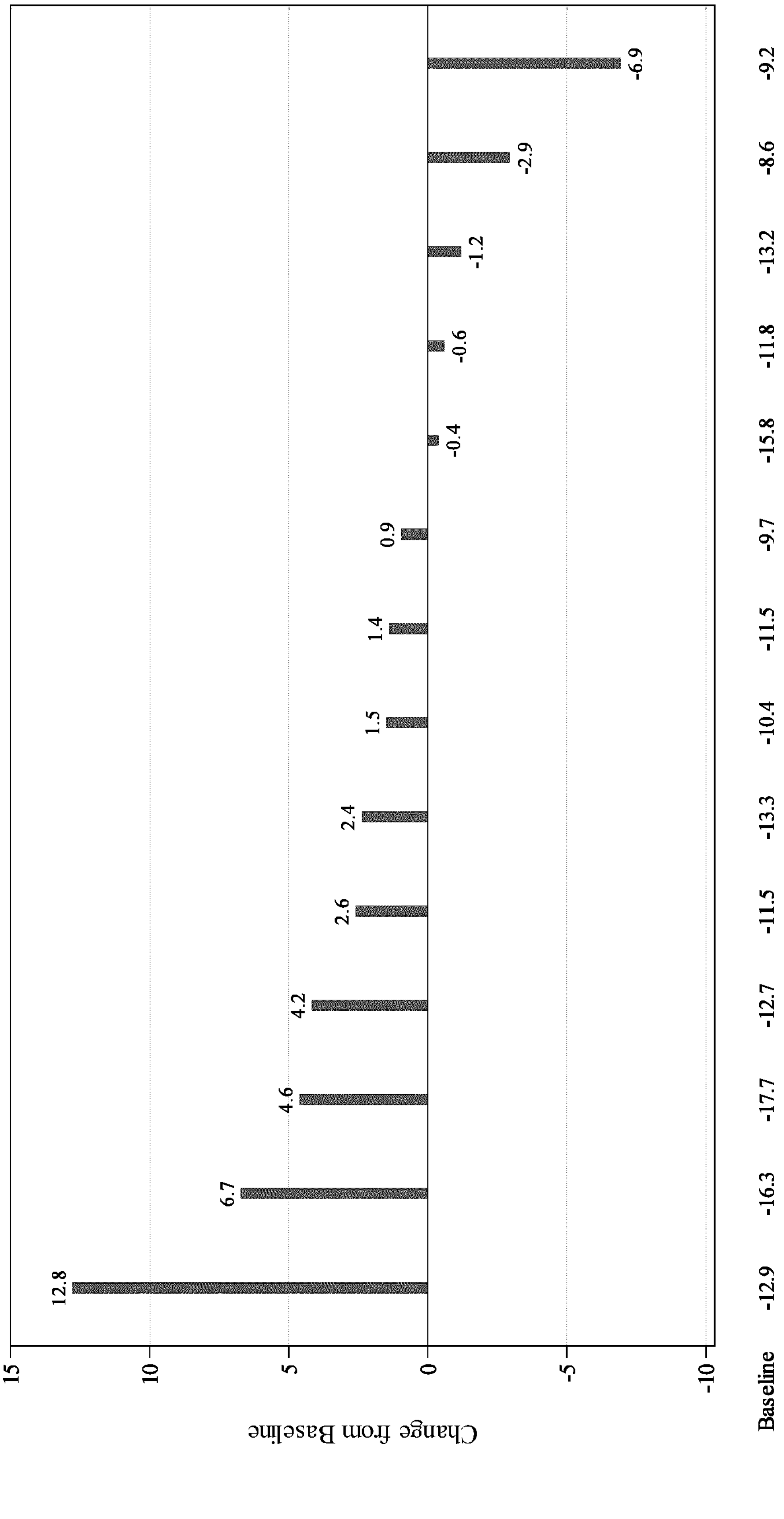


**Fig. 10**

Waterfall Plot of Change from Baseline at 1 Year in Cardiac Fibrosis Score

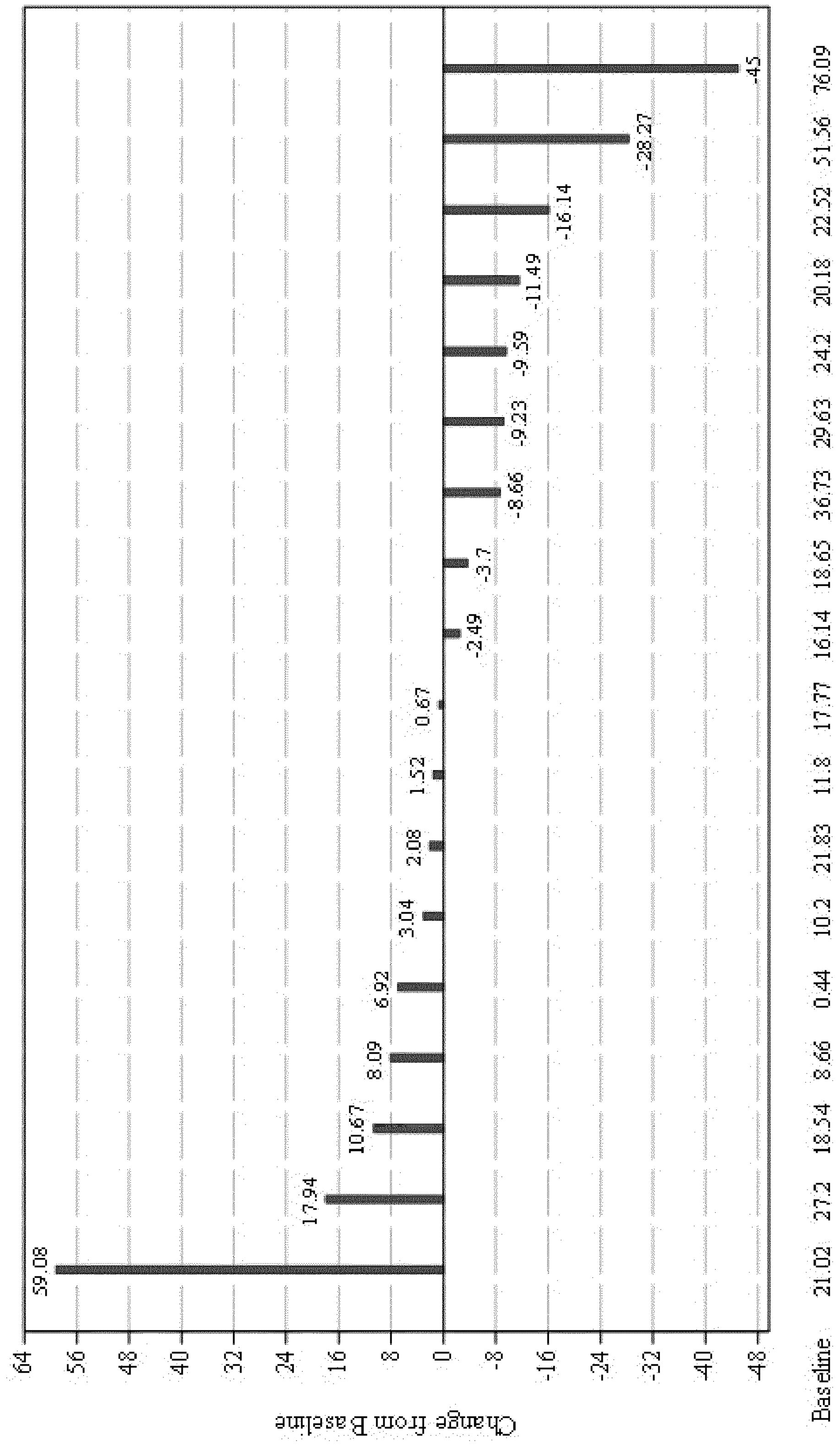


Fig. 11

**Change From Baseline to Year 1 in LVEF (%) Versus Change in Cardiac Fibrosis Score MLGE (G) at 1 Year**

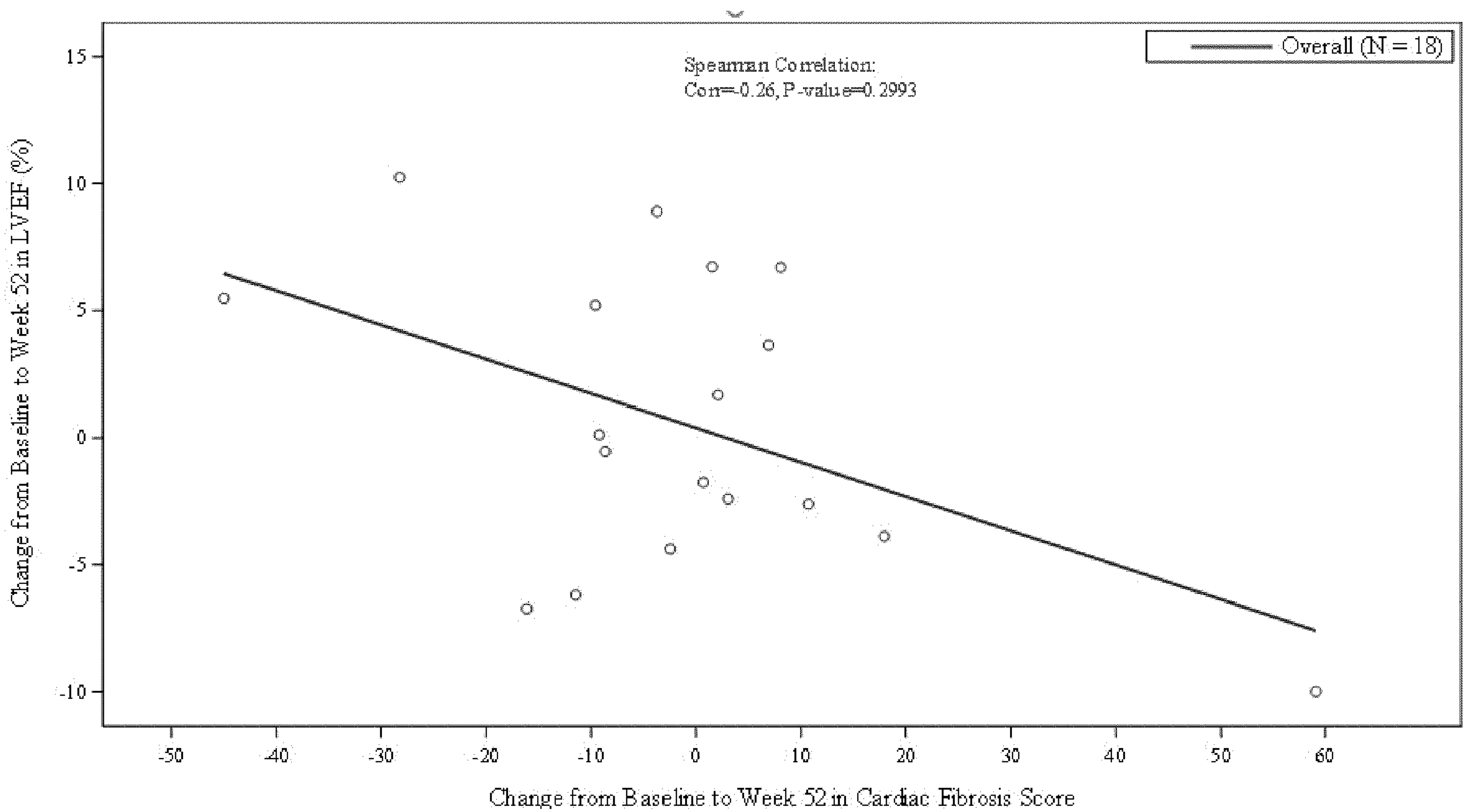


**Fig. 12**

# Waterfall Plot of Change from Baseline at Week 48 in Grip Strength (Dominant Hand) (Newton)

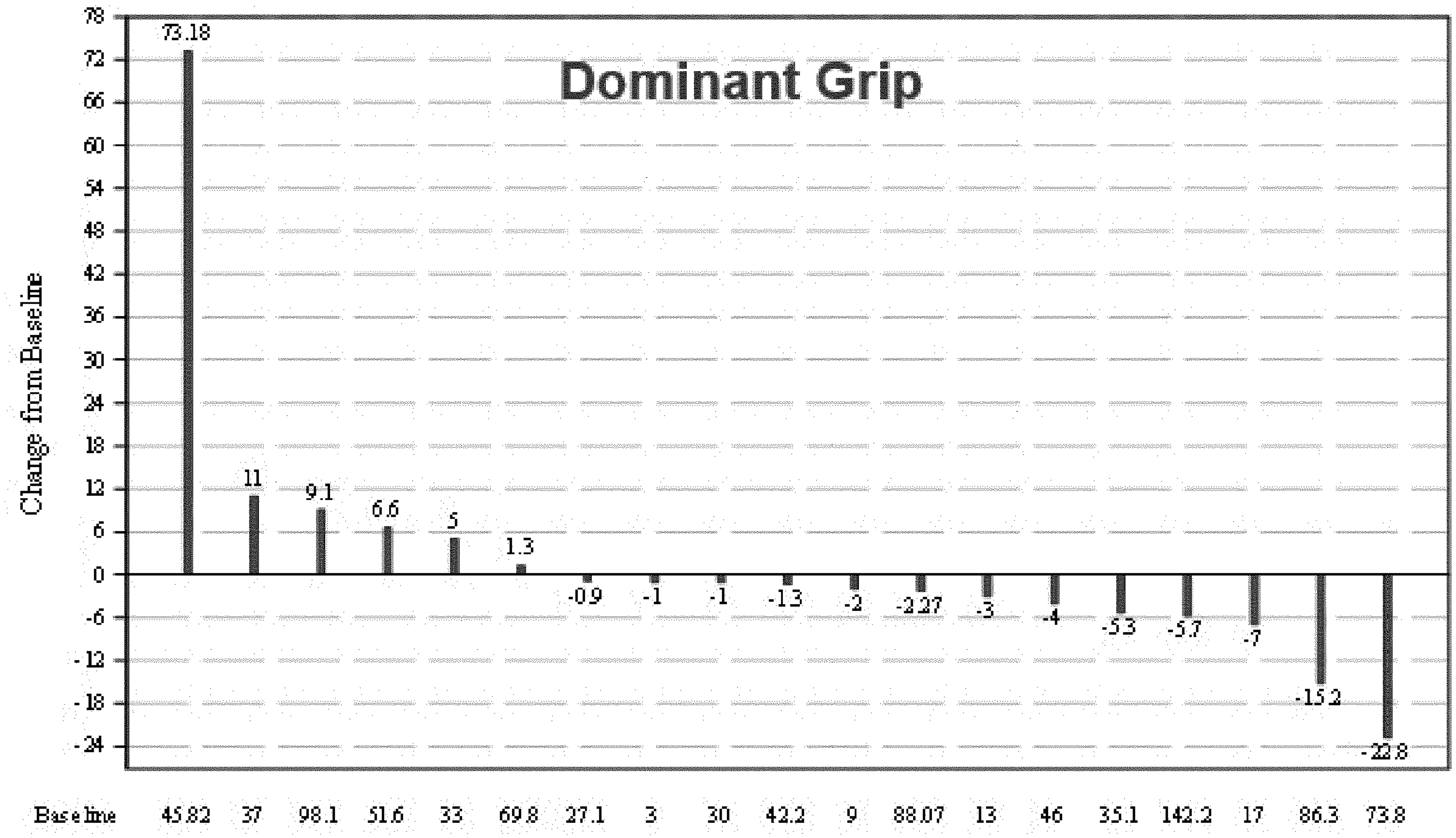


Fig. 13 A

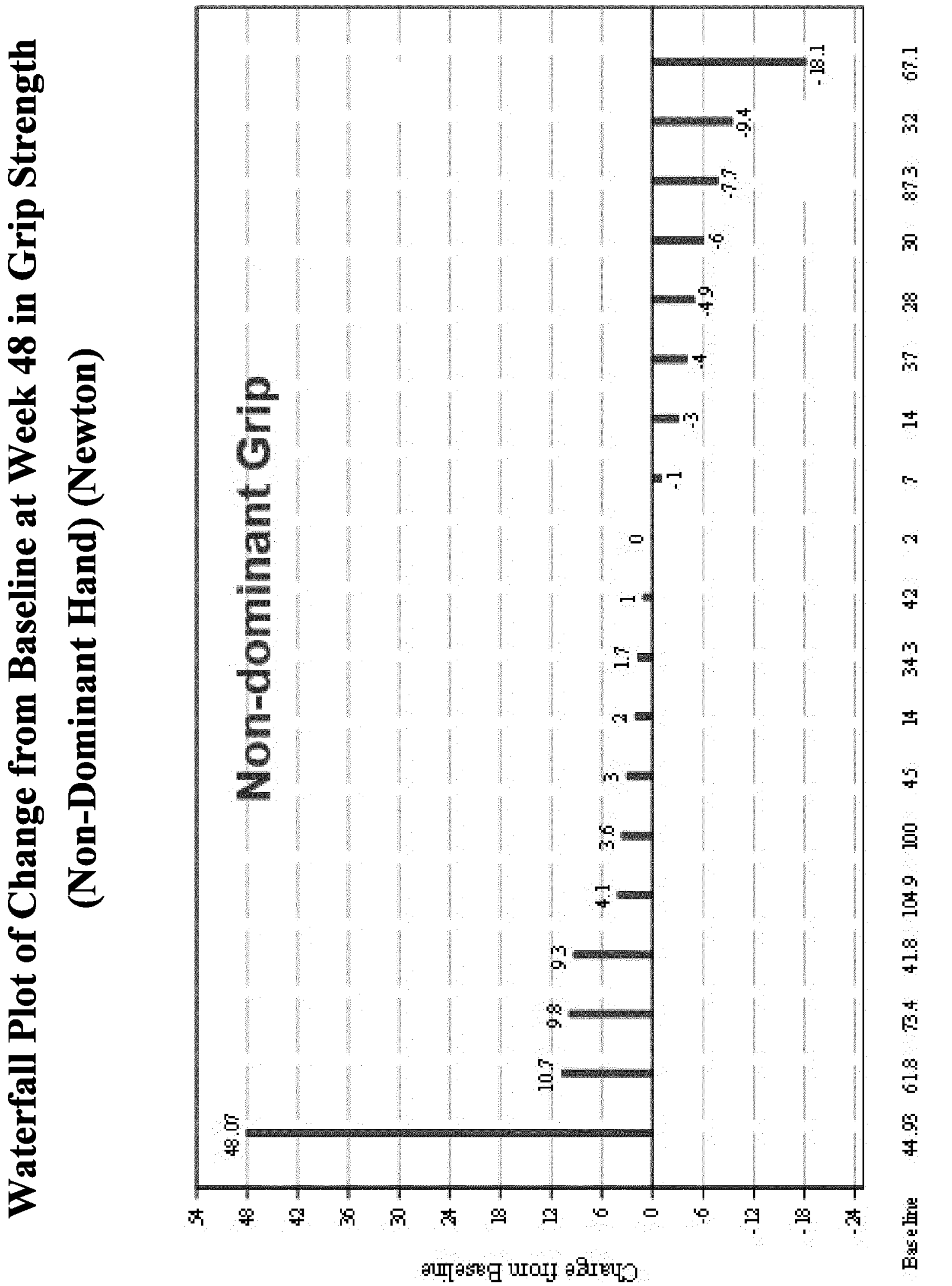
Waterfall Plot of Change from Baseline at Week 48 in Grip Strength
(Non-Dominant Hand) (Newton)
Non-dominant Grip
Change from Baseline
54
48
42
36
30
24
18
12
6
0
-6
-12
-18
-24
Baseline
44.93
61.8
73.4
41.8
104.9
100
45
14
34.3
42
2
7
14
37
28
30
87.3
32
67.1
48.07
10.7
9.8
9.3
4.1
3.6
3
2
1.7
1
0
-1
-3
-4
-4.9
-6
-7.7
-9.4
-18.1

Fig. 13 B

## Bar Plot of LS Mean Change from Baseline at 1 Year in Grip Strength (Newton)

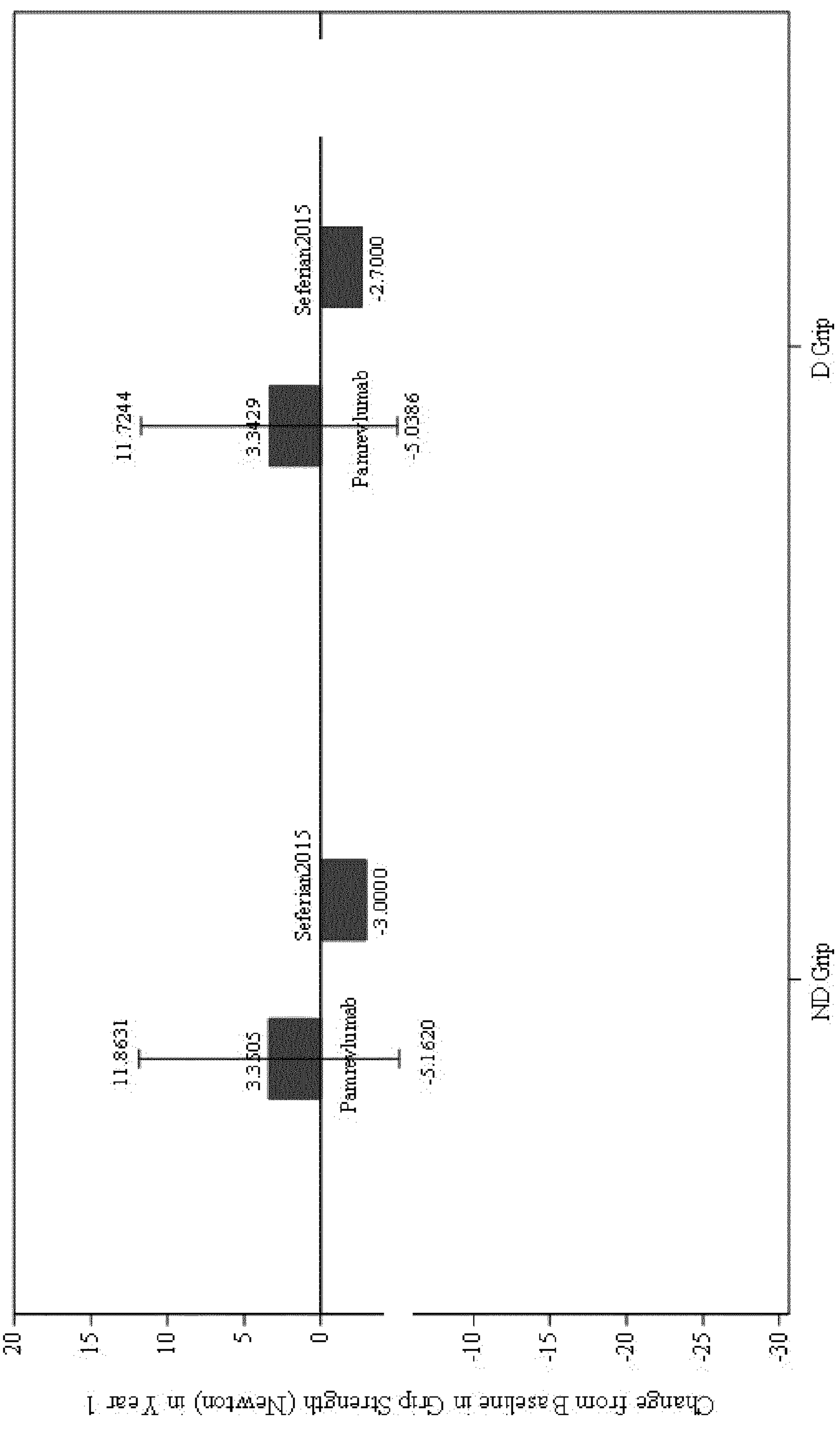


Fig. 14

**Waterfall Plot of Change from Baseline at Week 48 in PUL Score**

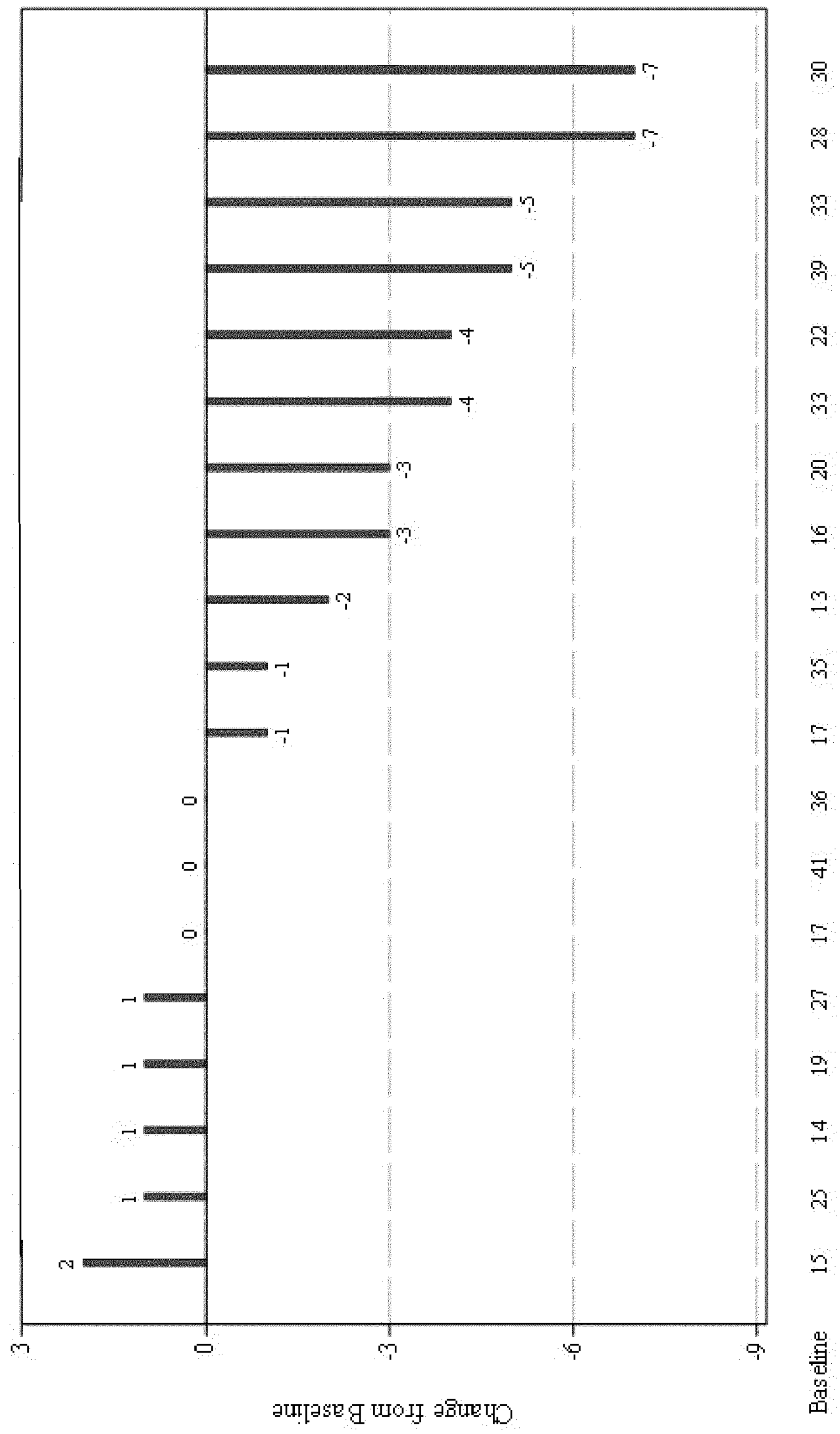


**Fig. 15**

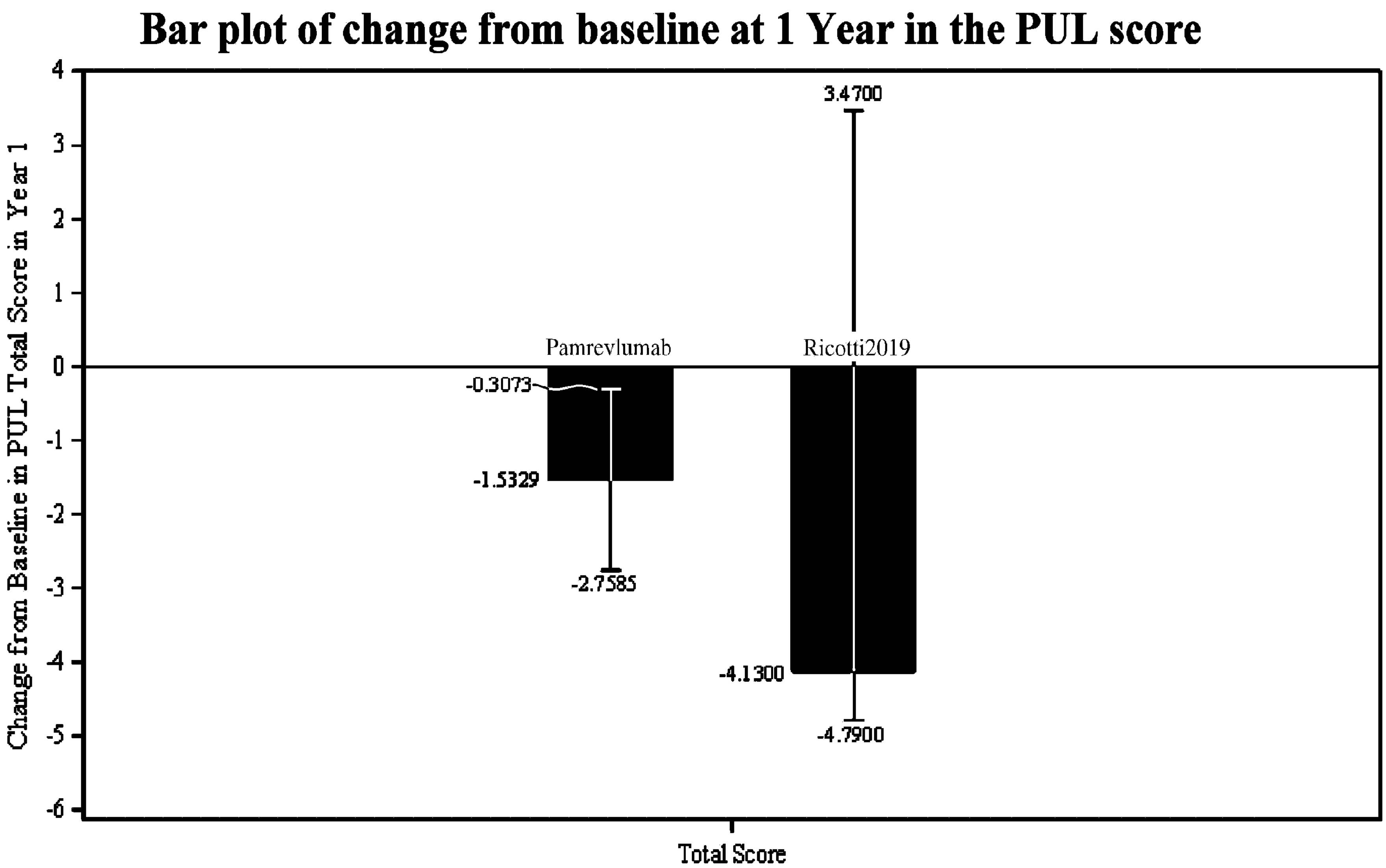
Bar plot of change from baseline at 1 Year in the PUL score
Change from Baseline in PUL Total Score in Year 1
4
3
2
1
0
-1
-2
-3
-4
-5
-6
Pamrevlumab
Ricotti2019
3.4700
-0.3073
-1.5329
-2.7585
-4.1300
-4.7900
Total Score

Fig. 16

**Waterfall Plot of Change from Baseline at 1 Year in Upper Arm T2-Mapping Score**

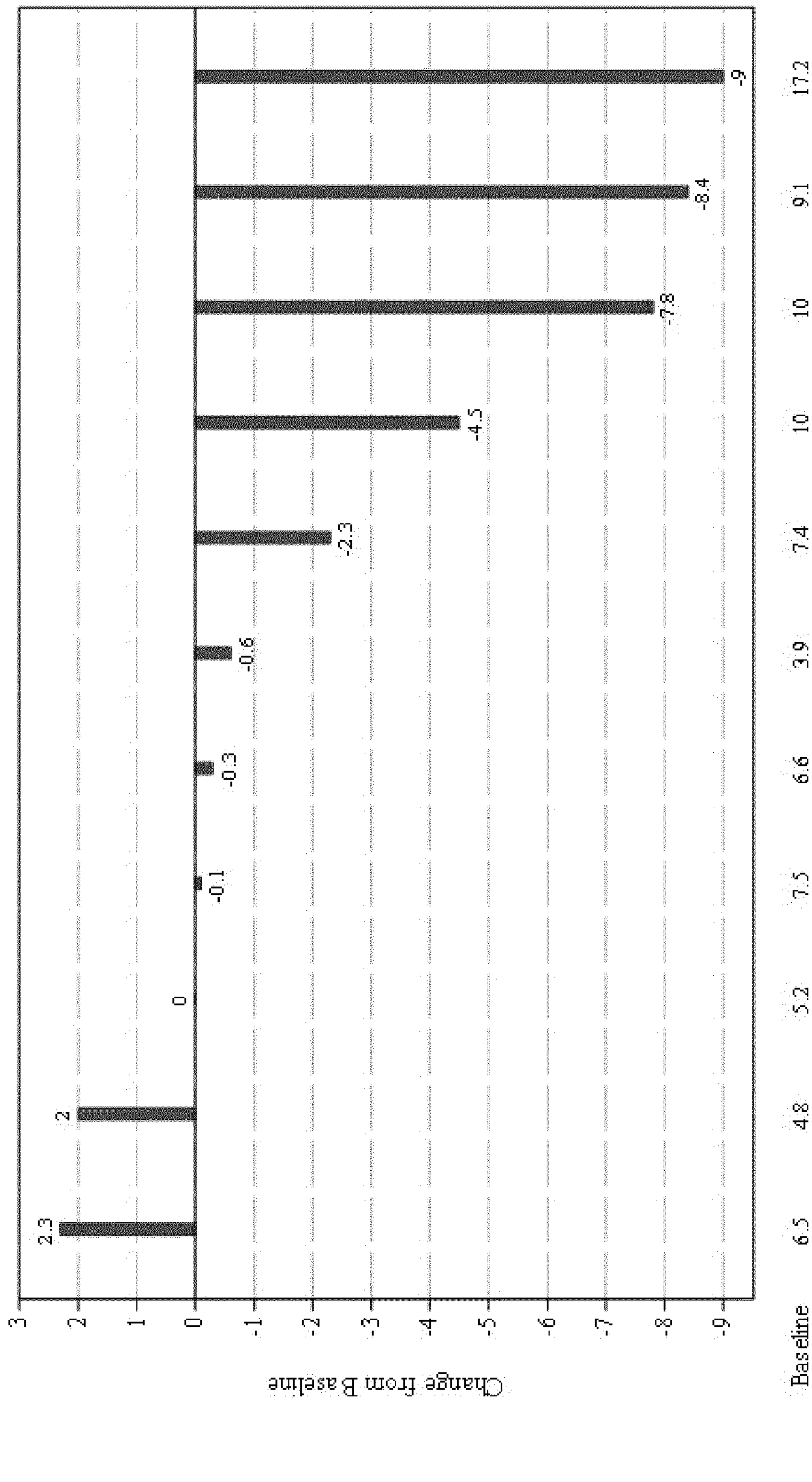


**Fig. 17**

**Fig. 18** **Bar Plot of LS Mean Change from Baseline in Upper Arm Fat Fraction (%) at 1 Year**

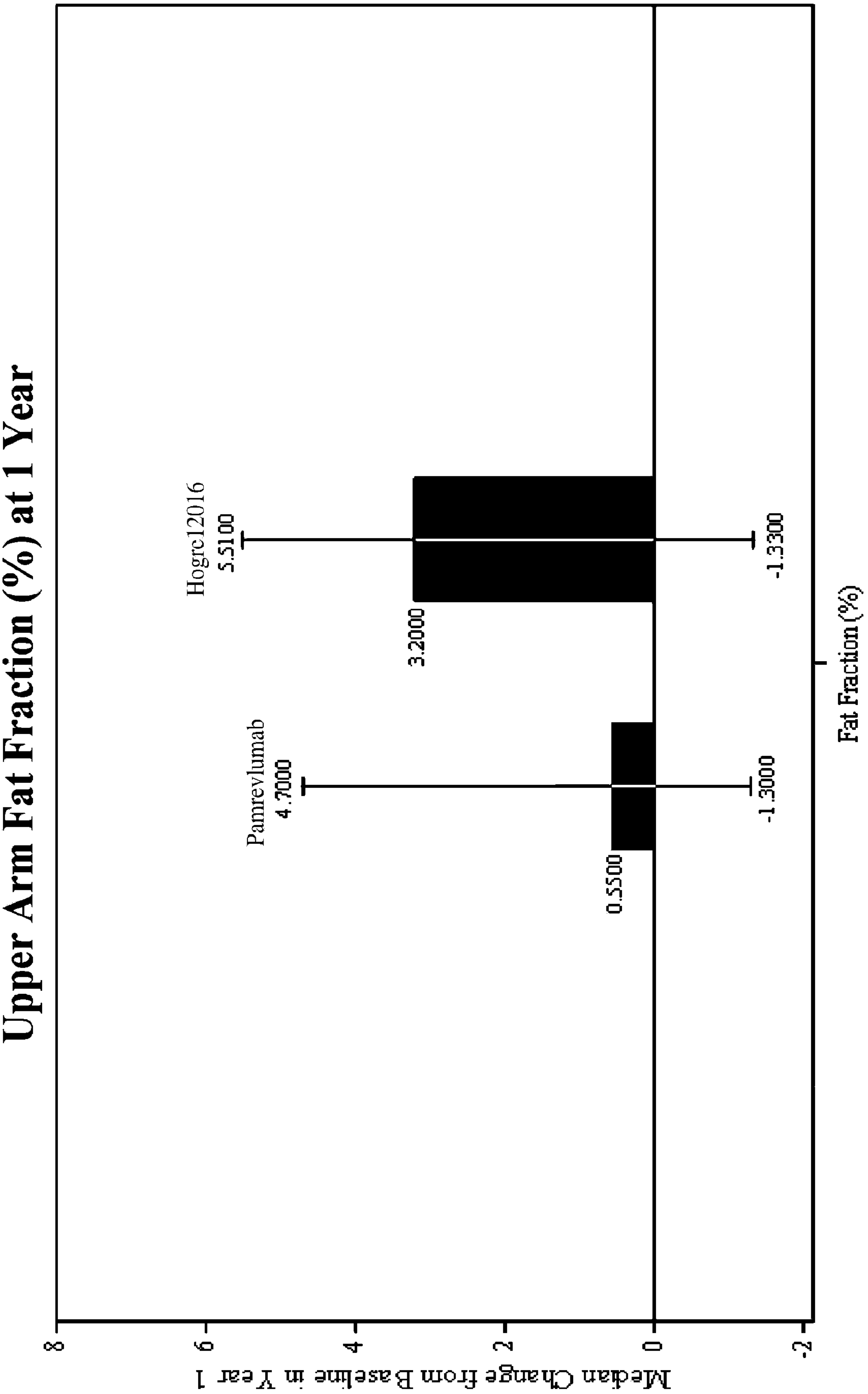

**Change from Baseline to 1 Year in Upper Arm Biceps Brachii T2-Mapping Versus Change in PUL Total Score in 1 Year**

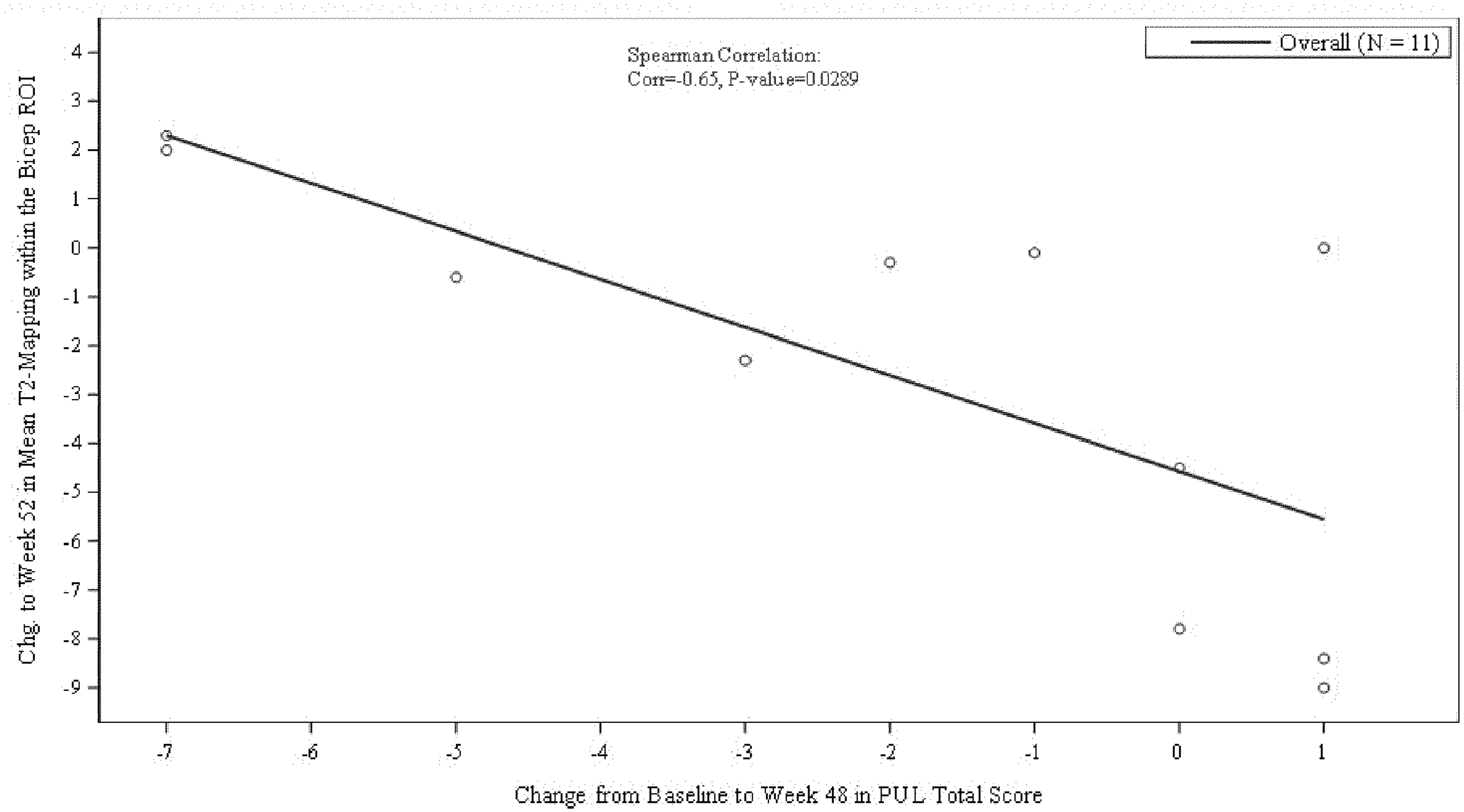


**Fig. 19**

Europäisches Patentamt
European Patent Office
Office européen des brevets

# EUROPEAN SEARCH REPORT

Application Number
EP 20 17 6176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/100193 A1 (FIBROGEN INC [US]) 15 June 2017 (2017-06-15) * paragraph [0002] * * paragraph [0006] * * paragraph [0010] * * paragraph [0011] * * paragraph [0038] * ----- | 1-17 | INV. A61K39/395 A61P9/00 A61P11/00 A61P21/00 |
| X | US 2014/271573 A1 (BRANDAN ENRIQUE [CL] ET AL) 18 September 2014 (2014-09-18) * paragraph [0047]; claims 1, 3 * * paragraph [0051] * ----- | 1-17 | |
| X | WO 2011/056234 A1 (FIBROGEN INC [US]; LIPSON KENNETH E [US]) 12 May 2011 (2011-05-12) * abstract * * the whole document * ----- | 1-17 | |
| X | FIBROGEN: "Press Release: FibroGen Announces Promising Preliminary Data from an Open-label Phase 2 Study to Evaluate the Safety and Efficacy of FG-3019 in Individuals with Idiopathic Pulmonary Fibrosis (IPF) and Provides Program Update", INTERNET CITATION, 3 May 2012 (2012-05-03), pages 1-3, XP002747981, Retrieved from the Internet: URL:http://investor.fibrogen.com/phoenix.zhtml?c=253783&p=irol-newsArticle_Print&ID=1983434 [retrieved on 2015-10-22] * page 3, paragraph 1st * ----- | 1-17 | TECHNICAL FIELDS SEARCHED (IPC) A61K A61P |

-/--

The present search report has been drawn up for all claims

1

| Place of search | Date of completion of the search | Examiner |
|---|---|---|
| Munich | 12 October 2020 | Baumgärtner, Heike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 17 6176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LUCAS R. SMITH ET AL: "Regulation of fibrosis in muscular dystrophy", MATRIX BIOLOGY, vol. 68-69, 1 August 2018 (2018-08-01), pages 602-615, XP055738690, NL ISSN: 0945-053X, DOI: 10.1016/j.matbio.2018.01.014 * the whole document * ----- | 1-17 | |
| | | | TECHNICAL FIELDS SEARCHED (IPC) |

1 The present search report has been drawn up for all claims

| Place of search | Date of completion of the search | Examiner |
|---|---|---|
| Munich | 12 October 2020 | Baumgärtner, Heike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 20 17 6176

This annex lists the patent family members relating to the patent documents cited in the above-mentioned European search report.
The members are as contained in the European Patent Office EDP file on
The European Patent Office is in no way liable for these particulars which are merely given for the purpose of information.

12-10-2020

| Patent document cited in search report | Publication date | Patent family member(s) | Publication date |
|---|---|---|---|
| WO 2017100193 A1 | 15-06-2017 | EP 3386544 A1 | 17-10-2018 |
| | | US 2019002547 A1 | 03-01-2019 |
| | | WO 2017100193 A1 | 15-06-2017 |
| US 2014271573 A1 | 18-09-2014 | US 2014271573 A1 | 18-09-2014 |
| | | WO 2015135089 A1 | 17-09-2015 |
| WO 2011056234 A1 | 12-05-2011 | US 2012244169 A1 | 27-09-2012 |
| | | WO 2011056234 A1 | 12-05-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62863143
- US 62852227
- US 7405274 B
- US 4816567 A
- WO 199824893 A
- WO 199634096 A
- WO 199633735 A
- WO 199110741 A
- US 5545807 A
- US 5545806 A
- US 5569825 A
- US 5625126 A
- US 5633425 A
- US 5661016 A
- US 6982321 B
- US 7087409 B
- US 6075181 A
- US 6150584 A
- US 5408040 A
- US 1998016423 W
- US 1999029652 W
- WO 9933878 A
- US 9587015 B
- WO 2013108869 A
- US 7871617 B
- US 2010044549 W
- US 5539082 A
- US 2010022759 W
- US 2010023397 W
- US 2009061913 W
- US 2009058013 W
- US 2009039557 W
- US 2009066863 W
- US 2009033373 W
- US 2009039438 W
- US 2009037686 W
- US 5830653 A
- WO 9813526 A
- US 5013830 A
- US 5214135 A
- US 5525719 A
- WO 9203568 A
- US 5276019 A
- US 5264423 A
- US 1996008140 W
- US 1999026189 W
- US 2002038618 W
- US 6358741 B
- US 6965025 B
- US 7462602 B
- US 20080070856
- US 20080176964
- US 8802839 B
- US 0238618 W
- US 2009054973 W
- US 2009054974 W
- US 2009054975 W
- US 2009054976 W
- US 2012023620 W
- US 364547
- US 7056704 B
- US 7078196 B
- US 7459547 B
- US 7691995 B
- US 7691997 B
- US 1996012251 W


### Non-patent literature cited in the description


- **MAH et al.** *Neuromuscul Disord,* 2014, vol. 24 (6), 482-491
- **ANDREWS et al.** *Adolesc Health Med Ther.,* 2018, vol. 9, 53-63
- **MCKIM et al.** *Arch Phys Med Rehabil.,* 2012, vol. 93 (7), 1117-1122
- **HENRICSON et al.** *Muscle Nerve.,* 2013, vol. 48 (1), 55-67
- **MAYER et al.** *Pediatr Pulmonol.,* 2015, vol. 50 (5), 487-494
- **FINDER et al.** *Am J Respir Crit Care Med.,* 2017, vol. 196 (4), 512-519
- **BARBER BJ et al.** *J Pediatr.,* October 2013, vol. 163 (4), 1080-4
- **TANDON et al.** *J Am Heart Assoc.,* 2015, vol. 4 (4), 3001338
- **SCHRAM et al.** *JAm Coll Cardiol.,* 2013, vol. 61 (9), 948-954
- **RICOTTI et al.** *Neuromuscul Disord.,* 2019, vol. 29 (4), 261-268
- **MEIER et al.** *Neuromuscl Disord.,* 2016, vol. 27, 307-314
- **MCDONALD et al.** *Lancet.,* 2018, vol. 391 (10119), 451-461

- **SEFERIAN et al.** *PLoS One.,* 2015, vol. 10 (2), e0113999
- **HOGREL JY et al.** *Neurology.,* 2016, vol. 86 (11), 1022-30
- Remington's Pharmaceutical Sciences. Mack Publishing Co, 1990
- Methods In Enzymology. Academic Press, Inc,
- Handbook of Experimental Immunology. Blackwell Scientific Publications, 1986, vol. I-IV
- Molecular Cloning: A Laboratory Manual. Cold Spring Harbor Laboratory Press, 1989, vol. I-III
- Short Protocols in Molecular Biology. John Wiley & Sons, 1999
- Molecular Biology Techniques: An Intensive Laboratory Course. Academic Press, 1998
- PCR (Introduction to Biotechniques Series),. Springer Verlag, 1997
- **MOXLEY et al.** *Neurology.,* 2005, vol. 64, 13-20
- **HOR et al.** *J AM Coll Cardiol.,* 07 April 2009, vol. 53 (14), 1204-1210
- **DIAZ-MANERA et al.** *Acta Myolgica.,* 2015, vol. 34, 95-108
- **KOHLER G ; MILSTEIN C.** *Nature,* 1975, vol. 256, 495-497
- **HARLOW E et al.** Antibodies: A Laboratory Manual. Cold Spring Harbor Laboratory Press, 1988
- **CLACKSON T et al.** *Nature,* 1991, vol. 352, 624-628
- **MARKS JD et al.** *J Mol Biol,* 1992, vol. 222, 581-597
- **LEE V et al.** *J Immunol Methods,* 2004, vol. 284 (1-2), 119-132
- **JAKOBOVITS A et al.** *Proc Natl Acad Sci USA,* 1993, vol. 90, 2551
- **MORRISON S et al.** *Proc Natl Acad Sci USA,* 1984, vol. 81, 6851-6855
- **JONES TA et al.** *Nature,* 1986, vol. 321, 522-525
- **RIECHMANN L et al.** *Nature,* 1988, vol. 332, 323-329
- **HOOGENBOOM HR ; WINTER G.** *J. Mol. Biol.,* 1992, vol. 227, 381
- **MARKS JD et al.** *J. Mol. Biol.,* 1991, vol. 222, 581
- **BOERNER R et al.** *J. Immunol.,* 1991, vol. 147 (1), 86-95
- **LI J et al.** *Proc. Natl. Acad. Sci. USA,* 2006, vol. 103, 3557-3562
- **MYZITHRAS et al.** *Bioanalysis.,* 01 March 2018, vol. 10 (6), 397-406
- *CHEMICAL ABSTRACTS,* 946415-13-0
- **HOLLIGER P et al.** *Proc Natl Acad Sci USA,* 1993, vol. 90, 6444-6448
- **ZAPATA G et al.** *Protein Eng,* 1995, vol. 8 (10), 1057-1062
- **BIRD KD et al.** *Science,* 1988, vol. 242, 423-426
- **GHAHOUDI MA et al.** *FEBS Lett.,* 1997, vol. 414, 521-526
- **WARD ES et al.** *Nature,* 1989, vol. 341, 544-546
- **LIPOVŠEK D.** *Protein Eng Des Sel,* 2010, vol. 24, 3-9
- **NORD K et al.** *Nat Biotechnol.,* 1997, vol. 15, 772-777
- **AMSTUTZ P.** *Protein Eng Des Sel.,* 2006, vol. 19, 219-229
- **BESTE G et al.** *Proc Natl Acad Sci USA.,* 1999, vol. 5, 1898-1903
- **SWAYZE E ; BHAT B.** Antisense Drug Technology Principles, Strategies. CRC Press, 2008, 144-182
- **KOSHKIN AA et al.** *Tetrahedron,* 1998, vol. 54, 3607-3630
- **SINGH SK et al.** *Chem. Commun,* 1998, vol. 4, 455-456
- **SUMMERTON J ; WELLER D.** *Antisense Nucleic Acid Drug Dev,* 1997, vol. 7, 187-195
- **NIELSEN PE et al.** *Science,* 1991, vol. 254, 1497-1500
- **HERDEWIJN P.** *Antisense Nucleic Acid Drug Dev,* 2000, vol. 10, 297-310
- **SANGHVI Y S et al.** *Nucleic Acids Res,* 1993, vol. 21, 3197-3203
- **STEC W et al.** *JAm Chem Soc,* 1984, vol. 106, 6077-6079
- **STEC W et al.** *J Org. Chem,* 1985, vol. 50, 3908-3913
- **STEC W et al.** *J Chromatog,* 1985, vol. 326, 263-280
- **LAPLANCHE JL et al.** *Nucl Acid Res,* 1986, vol. 26, 251-60
- **FASMAN G D.** Practical Handbook of Biochemistry and Molecular Biology. CRC Press, 1989
- **KOTHAPALLI D et al.** *Cell Growth Differ,* 1997, vol. 8, 61-68
- **SHIMO T et al.** *J Biochem,* 1998, vol. 124, 130-140
- **UCHIO K et al.** *Wound Repair Regen,* 2004, vol. 12, 60-66
- **GUHA M et al.** *FASEB J,* 2007, vol. 21, 3355-3368
- Remington's Pharmaceutical Sciences. 2000
- The Pharmacological Basis of Therapeutics. 2001
- Handbook of Pharmaceutical Additives. Synapse Information Resources, Inc, 2002
- **CONNOLLY et al.** *Muscle Nerve,* 2015, vol. 51 (4), 522-532
- **MCDONALD et al.** *Parent Project Muscular Dystrophy Pulmonary Endpoints Workshop,* 14 April 2016

- **BIRNKRANT et al.** *Lancet Neurol.,* 2018, vol. 17 (4), 347-361
- **MAYHEW et al.** *Dev Med Child Neurol.,* 2013, vol. 55 (11), 1038-45
- **PANE M et al.** *Neuromuscul Disord.,* 2014, vol. 24 (3), 201-6